# EUROPEAN PATENT SPECIFICATION

(11) **EP 4 077 371 B1**
(45) Date of publication and mention of the grant of the patent: **10.01.2024**
(21) Application number: 20829882.8
(22) Date of filing: 16.12.2020
(51) Int. Cl.: C07K 16/00, C07H 5/10, C12N 5/00, C12P 21/00, C12R 1/91

(54) **METHODS AND COMPOSITIONS FOR MODULATING THE GLYCOSYLATION PROFILE OF PROTEINS**
VERFAHREN UND ZUSAMMENSETZUNGEN ZUM MODULIEREN DES GLYKOSYLIERUNGSPROFILS VON PROTEINEN
PROCÉDÉS ET COMPOSITIONS POUR MODULER LE PROFIL DE GLYCOSYLATION DE PROTÉINES

(30) Priority: 19.12.2019 EP 19218172
(43) Date of publication of application: 26.10.2022
(73) Proprietor: Merck Patent GmbH, 64293 Darmstadt (DE)
(72) Inventor: ZIMMER, Aline, 64293 Darmstadt (DE); EHRET, Janike, 64293 Darmstadt (DE); ZIMMERMANN, Martina, 64293 Darmstadt (DE)
(74) Representative: Merck Patent Association
(86) International application number: PCT/EP2020/086410
(87) International publication number: WO 2021/122738

(56) References cited:
- WO-A1-2019/191150
- WO-A2-2009/135181
- WO-A2-2012/019165
- WO-A2-2014/130613
- ZIMMERMANN MARTINA ET AL: "Impact of Acetylated and Non-Acetylated Fucose Analogues on IgG Glycosylation", ANTIBODIES, vol. 8, no. 1, 10 January 2019 (2019-01-10), page 9, XP055787067, CH ISSN: 2073-4468, DOI: 10.3390/antib8010009
- HASHIMOTO HIRONOBU ET AL: "Synthesis of 5-Thio-L-Fucose and its Inhibitory Effect on Fucosidase", JOURNAL OF CARBOHYDRATE CHEMISTRY, vol. 9, no. 5, 1 January 1990 (1990-01-01) , pages 683-694, XP055786947, UK ISSN: 0732-8303, DOI: 10.1080/07328309008543863 Retrieved from the Internet: URL:http://dx.doi.org/10.1080/073283090085 43863>
- ZANDBERG WESLEY F. ET AL: "Metabolic Inhibition of Sialyl-Lewis X Biosynthesis by 5-Thiofucose Remodels the Cell Surface and Impairs Selectin-Mediated Cell Adhesion*", JOURNAL OF BIOLOGICAL CHEMISTRY, vol. 287, no. 47, 1 November 2012 (2012-11-01), pages 40021-40030, XP055787441, ISSN: 0021-9258, DOI: 10.1074/jbc.M112.403568
- ZIMMERMANN MARTINA ET AL: "Use of 5-Thio-L-Fucose to modulate binding affinity of therapeutic proteins", BIOTECHNOLOGY AND BIOENGINEERING, 27 January 2021 (2021-01-27), XP055787063, US ISSN: 0006-3592, DOI: 10.1002/bit.27695 Retrieved from the Internet: URL:https://onlinelibrary.wiley.com/doi/fu ll-xml/10.1002/bit.27695>

## Description

The present invention relates to cell culture media comprising 5-Thio-L-fucose and the use of 5-Thio-L-fucose for modulating the glycosylation profile of antibodies or other Fc containing proteins and thereby modulating the binding affinity of said proteins.

Recombinant proteins like therapeutic antibodies and Fc-fusion proteins are commonly produced in mammalian cell lines.

Monoclonal antibodies and Fc-fusion proteins produced in mammalian host cells can have a variety of post-translational modifications, including glycosylation. Monoclonal antibodies, such as IgGs, typically have an N-linked glycosylation site at asparagine 297 (Asn297) of each heavy chain (two per intact antibody). The glycans attached to Asn297 on antibodies are typically complex biantennary structures with very low or no bisecting N-acetylglucosamine (bisecting GlcNAc) with low amounts of terminal sialic acid and variable amounts of galactose. The glycans also usually have high levels of core fucosylation. Reduction of core fucosylation in antibodies has been shown to alter Fc effector functions, in particular Fc gamma receptor binding and ADCC activity. This observation has led to interest in the engineering of cell lines that produce antibodies with reduced core fucosylation.

Key for some antibody-based immunotherapy is the binding of the antibody Fc part to cell surface receptors called Fcγ receptors (FcγR). Depending on the intracytoplasmic domain, FcγR trigger different immune responses. Common are immunoreceptor tyrosine-based activation motifs (ITAMs) for activating receptors (FcγRI, FcγRIIa, FcγRIIc, FcγRIIIa and FcγRIIIb) and immunoreceptor tyrosine-based inhibition motifs (ITIMs) for inhibitory receptors like FcγRIIb [Lu J and Sun PD, Structural mechanism of high affinity FcgammaRI recognition of immunoglobulin G. Immunol Rev 2015, 268, 192-200]. Furthermore, receptors can be classified into high-affinity FcγRI (CD64) as well as low-affinity FcγRII (CD32) and FcγRIII (CD16). Bruhns *et al.* described the binding of different receptor classes to all known antibody subclasses. IgG1 are reported to bind all classes of receptors with different affinities. Increasing affinities of IgG1 were observed for FcγRIIb, FcγRIIIb, FcyRIIIa and FcγRIIa with the highest affinity for FcγRI [Bruhns P, Iannascoli B, England P, Mancardi DA, Fernandez N, Jorieux S, and Daeron M, Specificity and affinity of human Fcgamma receptors and their polymorphic variants for human IgG subclasses. Blood 2009, 113, 3716-25]

Two major Fc effector functions are antibody-dependent cellular cytotoxicity (ADCC) and complement-dependent cytotoxicity (CDC). Natural killer (NK) cells expressing FcγRIIIa are responsible for the ADCC activity. In general, the receptor affinity and thereby biological activity of an antibody depends on post-translational modifications like glycosylation of the antibody [Harris RJ, Chin ET, Macchi F, Keck RG, Shyong B-J, Ling VT, Cordoba AJ, Marian M, Sinclair D, Battersby JE, and Jones AJS, Analytical Characterization of Monoclonal Antibodies: Linking Structure to Function. In Current Trends in Monoclonal Antibody Development and Manufacturing, ed.; Shire SJ, et al.; Springer New York: New York, NY, 2010, 193-205].

The antibody presents a distinct N-linked glycosylation at position 297 (Asn297) on each heavy chain in the Fc-part. The presence of core-fucosylation on Fc part of the recombinant protein is known to reduce the ADCC response due to steric hindrance of fucose [Mizushima T, Yagi H, Takemoto E, Shibata-Koyama M, Isoda Y, Iida S, Masuda K, Satoh M, and Kato K, Structural basis for improved efficacy of therapeutic antibodies on defucosylation of their Fc glycans. Genes to cells 2011, 16, 1071-1080]. Therefore, reduction of core-fucosylation leads to enhanced ADCC. This was verified by *in vivo* studies of afucosylated anti-EGFR and anti-CS1 antibodies showing enhanced ADCC and anti-tumor activity when compared to their fucosylated counterparts. Gomathinayagam S, Laface D, Houston-Cummings NR, Mangadu R, Moore R, Shandil I, Sharkey N, Li H, Stadheim TA, and Zha D, In vivo anti-tumor efficacy of afucosylated anti-CS1 monoclonal antibody produced in glycoengineered Pichia pastoris. Journal of Biotechnology 2015, 208, 13-21. Concluding, modulation of the Fc glycosylation is a strategy to enhance biological activity of therapeutic antibodies.

Methods to reduce fucosylation include methods for engineering cell lines. Alternatives to engineering cell lines include the use of small molecule inhibitors that act on enzymes in the glycosylation pathway. Another option is the provision of small molecule fucose analogs for use in producing recombinant antibodies that have complex N-linked glycans, but have reduced fucosylation. This has for example been suggested in WO 09135181.

But still modulation of the glycosylation pattern and thus the modulation of the biological activity of therapeutic proteins is open for improvement.

It has been discovered that the addition of 5-Thio-L-fucose and/or certain derivatives thereof to cell culture media and feed results in a reduced fucosylation of the antibody / Fc containing proteins. The generated proteins exhibited an enhanced FcγRIIIa binding, which is correlated with an increased ADCC. Surprisingly, the use of 5-Thio-L-fucose and/or certain derivatives thereof led to a high incorporation of the fucose-analogue.

The present invention is therefore directed to a method of making an antibody or other Fc containing protein having reduced fucosylation, comprising:
culturing a host cell in a culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate, wherein the host cell expresses the antibody or other Fc containing protein having an Fc domain having at least one N-glycoside- linked sugar chain bound to the Fc domain through an N-acetylglucosamine of the reducing terminal of the sugar chain, and isolating the antibody or other Fc containing protein from the cells, wherein the antibody or other Fc containing protein has reduced fucosylation at the sugar chain compared to the antibody or other Fc containing protein from the same host cell cultured under the otherwise same culturing conditions but in the absence of 5-Thio-L-fucose and/or a derivative thereof, the derivative being selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate.

In a preferred embodiment the host cells are cultured in a cell culture medium comprising 5-Thio-L-fucose and/or partially or fully acetylated 5-Thio-L-fucose.

In a preferred embodiment the host cell is a Chinese hamster ovary cell.

In another preferred embodiment the host cell is cultured in a fed batch cell culture or a perfusion cell culture.

In a preferred embodiment the host cell is cultured by
- Filling into a bioreactor the host cells and an aqueous cell culture medium
- Incubating the cells in the bioreactor
- Continuously over whole time of the incubation of the cells in the bioreactor or once or several times within said incubation time adding a cell culture medium to the bioreactor, whereby the cell culture medium comprises 5-Thio-L-fucose.

Preferably the medium that is added has a pH below pH 8.5 and comprises 5-Thio-L-fucose in a concentration between 10 nmol/l and 100 mmol/l, preferably between 0.1 µmol/l and 10 mmol/l.

The present invention is further directed to a method for making a thiofucosylated antibody or other Fc containing protein comprising: culturing a host cell in a culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate, wherein the host cell expresses the antibody or other Fc containing protein having an Fc domain having at least one N-glycoside- linked sugar chain bound to the Fc domain through an N-acetylglucosamine of the reducing terminal of the sugar chain, and isolating the antibody or other Fc containing protein from the cells.

The present invention is further directed to a dry powder cell culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate.

In one embodiment, the cell culture medium is a feed medium.

In another embodiment the cell culture medium comprises 5-Thio-L-fucose and/or a derivative thereof in an amount that when being dissolved to provide a liquid medium the concentration of the 5-Thio-L-fucose and/or a derivative thereof in the liquid medium is between 10 nmol/l and 100 mmol/l, preferably between 0.1 µmol/l and 10 mmol/l.

The liquid medium, e.g. a feed medium or a perfusion medium, is typically designed such that the final concentration of 5-Thio-L-fucose and/or a derivative thereof in the cell culture is between 0.1 and 1mmol/l.

In one embodiment, the cell culture medium comprises at least one or more saccharide components, one or more amino acids, one or more vitamins or vitamin precursors, one or more salts, one or more buffer components, one or more co-factors and one or more nucleic acid components.

The present invention is further directed to a method for producing a cell culture medium according to the present invention by
a) mixing 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate with the other components of the cell culture medium
b) subjecting the mixture of step a) to milling

In a preferred embodiment step b) is performed in a pin mill, fitz mill or a jet mill.

In another preferred embodiment, the mixture from step a) is cooled to a temperature below 0°C prior to milling.
Figure 1 shows the structure similarity of L-Fucose and 5-Thio-L-Fucose with and without acetylation.
Figures 2 to 5 show the impact of 5-Thio-L-Fucose on cell performance using four different CHO cell lines producing three different IgG1 (A, B and C) and a fusion protein (D). Viable cell density (VCD) is visualized on the left and titer is visualized on the right. Control feed contains no supplements and DMSO control contains 1.17% DMSO which is the same concentration used as solvent for both peracetylated fucose-analogues. Further details see Example 1.
Fig 6 shows sugar structures (**A** fucosylated G0F, **B** thiofucosylated G0ThioF and **C** G1 fragmentation pattern). Details about Figure 6 can be found in Example 2.
Fig 7 shows the fluorescence signal obtained after separation of the released, labelled glycans using UPLC, showing a changed retention time of the peaks corresponding to thiofucosylated glycans compared to fucosylated glycans. Details about Figure 7 can be found in Example 2.
Fig 8 shows the impact of 5-Thio-L-Fucose on fucosylation profile using CHO cell lines producing (A) mAb1, (B) mAb2, (C) mAb3 and (D) a fusion protein. Details can be found in Example 3.
Fig 9 shows the accelerated effect of 5-Thio-L-Fucose during fed-batch experiments producing (A) mAb1 or (B) mAb3. Details can be found in Example 4.
Fig 10 shows the dose response of 5-Thio-L-Fucose on glycosylation profile. Further details can be found in Example 5.
Fig 11 shows the overlay plot of FcγRIIIa binding to mAb3 control (black) or binding to 5-Thio-L-Fucose treated mAb3 (grey) on day 12. Further details can be found in Example 6.

Cell culture media support and maintain the growth of cells in an artificial environment. Depending on the type of organism whose growth shall be supported, the cell culture media comprise a complex mixture of components, sometimes more than one hundred different components. The cell culture media required for the propagation of mammalian, insect or plant cells are typically much more complex than the media to support the growth of bacteria and yeasts. Chemically defined media often comprise but are not exclusively limited to amino acids, vitamins, metal salts, antioxidants, chelators, growth factors, buffers, hormones, and many more substances known to those expert in the art.

A cell culture medium according to the present invention is any mixture of components which maintains and/or supports the *in vitro* growth of cells. It might be a complex medium or a chemically defined medium. The cell culture medium can comprise all components necessary to maintain and/or support the *in vitro* growth of cells or only some components so that further components are added separately. Examples of cell culture media according to the present invention are full media which comprise all components necessary to maintain and/or support the *in vitro* growth of cells as well as media supplements or feeds. In a preferred embodiment the cell culture medium is a full medium, a perfusion medium or a feed medium. A full medium also called basal medium typically has a pH between 6.8 and 7.8. A feed medium preferably has a pH below 8.5, preferably between 6.5 and 8.5.

The cell culture medium might be a liquid medium or a dry powder medium.

Typically, the cell culture media according to the invention are used to maintain and/or support the growth of cells in a bioreactor.

A feed or feed medium is a cell culture medium which is not the basal medium that supports initial growth and production in a cell culture but the medium which is added at a later stage to prevent depletion of nutrients and sustains the production phase. A feed medium can have higher concentrations of some components compared to a basal culture medium. For example, some components, such as, for example, nutrients including amino acids or carbohydrates, may be present in the feed medium at about 5X, 6X, 7X, 8X, 9X, 10X, 12X, 14X, 16X, 20X, 30X, 50X, 100x, 200X, 400X, 600X, 800X, or even about 1000X of the concentrations in a basal medium.

A mammalian cell culture medium is a mixture of components which maintain and/or support the *in vitro* growth of mammalian cells. Examples of mammalian cells are human or animal cells, preferably CHO cells, COS cells, I VERO cells, BHK cells, AK-1 cells, SP2/0 cells, L5.1 cells, hybridoma cells or human cells.

Chemically defined cell culture media are cell culture media that do not comprise any chemically undefined substances. This means that the chemical composition of all the chemicals used in the media is known. The chemically defined media do not comprise any yeast, animal or plant tissues; they do not comprise feeder cells, serum, extracts or digests or other components which may contribute chemically poorly defined proteins to the media. Chemically undefined or poorly defined chemical components are those whose chemical composition and structure is not known, are present in varying composition or could only be defined with enormous experimental effort - comparable to the evaluation of the chemical composition and structure of a protein like albumin or casein.

A powdered cell culture medium or a dry powder medium is a cell culture medium typically resulting from a milling process, a lyophilisation process or a dry or wet granulation process. That means the powdered cell culture medium is a granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated. Dry powder media resulting from a milling or lyophilisation process typically have particle sizes below 0.5 mm, e.g. between 0.05 and 0.5 mm.

Dry powder media resulting from dry or wet granulation process, e.g. by spray drying, wet granulation or dry compaction, typically have particle sizes above 0.5 mm, e.g. between 0.5 and 5 mm. Dry compaction is typically done in a roll press. US 6,383,810 B2 discloses a method of producing an agglomerated eukaryotic cell culture medium powder. The method comprises wetting a dry powder cell culture medium with a solvent and then re-drying the moistened medium to obtain a dry agglomerated cell culture medium.

In one embodiment the dry powder media according to the present invention are produced by dry compaction.

Cells to be cultured with the media according to the present invention may be prokaryotic cells like bacterial cells or eukaryotic cells like plant or animal cells. Preferably the cells are mammalian cells. The cells can be normal cells, immortalized cells, diseased cells, transformed cells, mutant cells, somatic cells, non-human germ cells, stem cells, precursor cells or embryonic cells, any of which may be established or transformed cell lines or obtained from natural sources. A host cell is a cell incorporating a gene for expressing an antibody or another Fc containing protein having an Fc domain.

The size of a particle means the mean diameter of the particle. If the size of particles is given it means that at least 80%, preferably at least 90%, of the particles have the given particle size or are in the given particle size range. The particle diameter is determined by laser light scattering.

An inert atmosphere is generated by filling the respective container or apparatus with an inert gas. Suitable inert gases are noble gases like argon or preferably nitrogen. These inert gases are non-reactive and prevent undesirable chemical reactions from taking place. In the process according to the present invention, generating an inert atmosphere means that the concentration of oxygen is reduced below 10% (v/v) absolute, e.g. by introducing liquid nitrogen or nitrogen gas.

Different types of mills are known to a person skilled in the art.

A pin mill, also called centrifugal impact mill, pulverizes solids whereby protruding pins on high-speed rotating disks provide the breaking energy.

Pin mills are for example sold by Munson Machinery (USA), Premium Pulman (India) or Sturtevant (USA).

A jet mill uses compressed gas to accelerate the particles, causing them to impact against each other in the process chamber. Jet mills are e.g. sold by Sturtevant (USA) or PMT (Austria).

A fitz mill commercialized by Fitzpatrick (USA), uses a rotor with blades for milling.

A process that is run continuously is a process that is not run batchwise. If a milling process is run continuously it means that the media ingredients are permanently and steadily fed into the mill over a certain time.

The cell culture media, especially the full media, according to the present invention typically comprise at least one or more saccharide components, one or more amino acids, one or more vitamins or vitamin precursors, one or more salts, one or more buffer components, one or more co-factors and one or more nucleic acid components.

The media may also comprise sodium pyruvate, insulin, vegetable proteins, fatty acids and/or fatty acid derivatives and/or pluronic acid and/or surface active components like chemically prepared non-ionic surfactants. One example of a suitable non-ionic surfactant are difunctional block copolymer surfactants terminating in primary hydroxyl groups also called poloxamers, e.g. available under the trade name pluronic ^{®} from BASF, Germany.

Saccharide components are all mono- or di-saccharides, like glucose, galactose, ribose or fructose (examples of monosaccharides) or sucrose, lactose or maltose (examples of disaccharides).

Examples of amino acids according to the invention are tyrosine, the proteinogenic amino acids, especially the essential amino acids, leucine, isoleucine, lysine, methionine, phenylalanine, threonine, tryptophane and valine, as well as the non-proteinogenic amino acids like D-amino acids, preferably the L-amino acids.

Tyrosine means L- or D- tyrosine, preferably L-tyrosine.

Cysteine means L- or D-cysteine, preferably L-cysteine.

Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid), Vitamin D (Ergocalciferol, cholecalciferol), Vitamin E (Tocopherols, tocotrienols) and Vitamin K (phylloquinone, menaquinones). Vitamin precursors are also included.

Examples of salts are components comprising inorganic ions such as bicarbonate, calcium, chloride, magnesium, phosphate, potassium and sodium or trace elements such as Co, Cu, F, Fe, Mn, Mo, Ni, Se, Si, Ni, Bi, V and Zn. Examples are Copper(II) sulphate pentahydrate (CuSO₄·5H₂O), Sodium Chloride (NaCl), Calcium chloride (CaCl₂·2H₂O), Potassium chloride (KCl), Iron(II)sulphate, sodium phosphate monobasic anhydrous (NaH₂PO₄), Magnesium sulphate anhydrous (MgSO₄), sodium phosphate dibasic anhydrous (Na₂HPO₄), Magnesium chloride hexahydrate (MgCl₂·6H₂O), zinc sulphate heptahydrate.

Examples of buffers are CO₂/HCO₃ (carbonate), phosphate, HEPES, PIPES, ACES, BES, TES, MOPS and TRIS.

Examples of cofactors are thiamine derivatives, biotin, vitamin C, NAD/NADP, cobalamin, flavin mononucleotide and derivatives, glutathione, heme nucleotide phophates and derivatives.

Nucleic acid components, according to the present invention, are the nucleobases, like cytosine, guanine, adenine, thymine or uracil, the nucleosides like cytidine, uridine, adenosine, guanosine and thymidine, and the nucleotides like adenosine monophosphate or adenosine diphosphate or adenosine triphosphate.

Feed media may have a different composition compared to full media. They typically comprise amino acids, trace elements and vitamins. They might also comprise saccharide components but sometimes for production reasons the saccharide components are added in a separate feed.

A suitable feed medium might for example comprise one or more of the following compounds:
L-ASPARAGINE MONOHYDRATE
L-ISOLEUCINE
L-PHENYLALANINE
SODIUM L-GLUTAMATE MONOHYDRATE
L-LEUCINE
L-THREONINE
L-LYSINE MONOHYDROCHLORIDE
L-PROLINE
L-SERINE
L-ARGININE MONOHYDROCHLORIDE
L-HISTIDINE MONOHYDROCHLORIDE MONOHYDRATE
L-METHIONINE
L-VALINE
MONO-SODIUM-L-ASPARTATE-MONOHYDRATE
L-TRYPTOPHAN
CHOLINE CHLORIDE
MYO-INOSITOL
NICOTINAMIDE
CALCIUM-D(+) PANTOTHENATE
PYRIDOXINE HYDROCHLORIDE
THIAMINE CHLORIDE HYDROCHLORIDE
VITAMIN B12 (CYANOCOBALAMINE) MICRONIZED BIOTIN
FOLIC ACID
RIBOFLAVIN
MAGNESIUM SULFATE ANHYDROUS
COPPER(II) SULFATE PENTAHYDRATE
ZINC SULFATE HEPTAHYDRATE
1,4-DIAMINOBUTANE DIHYDRCHLORIDE
AMMONIUM HEPTAMOLYBDATE TETRAHYDRATE
CADMIUM SULFATE HYDRATE
MANGANESE(II) CHLORIDE TETRAHYDRATE
NICKEL(II) CHLORIDE HEXAHYDRATE
SODIUM META SILICATE
SODIUM METAVANADATE
TIN(II) CHLORIDE DIHYDRATE
SODIUM SELENITE (ABOUT 45% SE)
SODIUM DIHYDROGEN PHOSPHATE MONOHYDRATE
AMMONIUM IRON(III) CITRATE (ABOUT 18% FE)

Freezing according to the present invention means cooling to a temperature below 0°C.

The term "antibody" refers to (a) immunoglobulin polypeptides and immunologically active portions of immunoglobulin polypeptides, i.e., polypeptides of the immunoglobulin family, or fragments thereof, that contain an antigen binding site that immunospecifically binds to a specific antigen (e.g., CD70) and an Fc domain comprising a complex N-glycoside-linked sugar chain(s), or (b) conservatively substituted derivatives of such immunoglobulin polypeptides or fragments that immunospecifically bind to the antigen (e.g., CD70). Antibodies are generally described in, for example, Harlow and Lane, Antibodies: A Laboratory Manual (Cold Spring Harbor Laboratory Press, 1988).

An Fc containing protein is any protein comprising an Fc domain or region comprising a complex N- glycoside linked sugar chain.

The term "monoclonal antibody" refers to an antibody that is derived from a single cell clone, including any eukaryotic or prokaryotic cell clone, or a phage clone, and not the method by which it is produced. Thus, the term "monoclonal antibody" is not limited to antibodies produced through hybridoma technology.

The term "Fc region" refers to the constant region of an antibody, e.g., a C_{H}1-hinge- C_{H}2-C_{H}3 domain, optionally having a C_{H}4 domain, or a conservatively substituted derivative of such an Fc region.

The term "Fc domain" refers to the constant region domain of an antibody, e.g., a C_{H}1, hinge, C_{H}2, C_{H}3 or C_{H}4 domain, or a conservatively substituted derivative of such an Fc domain.

The term "Fab region" refers to the variable region of an antibody which binds to the antigen.

An "antigen" is a molecule to which an antibody, typically the antibody Fab domain, specifically binds.

The terms "specific binding" and "specifically binds" mean that the antibody or antibody derivative will bind, in a highly selective manner, with its corresponding target antigen and not with the multitude of other antigens. Typically, the antibody or other Fc containing protein binds with an affinity of at least about 1×10⁻⁷ M, and preferably 10⁻⁸ M to 10⁻⁹ M, 10⁻¹⁰ M, 10⁻¹¹ M, or 10⁻¹² M and binds to the predetermined antigen with an affinity that is at least two-fold greater than its affinity for binding to a non-specific antigen (e.g., BSA, casein) other than the predetermined antigen or a closely-related antigen.

The terms "inhibit" or "inhibition of" means to reduce by a measurable amount, or to prevent entirely, especially to reduce by a measurable amount, or to prevent entirely the activity of a specific enzyme catalyzing a reaction.

The term "afucosylation" or "afucosylated" refers to a glycan structure without fucose at the inner core-structure.

As used herein, "thiofucosylation" or "thiofucosylated" refers to a glycan structure comprising at least one thiofucose. Often in a "thiofucosylated" glycan structure fucose is substituted by thiofucose.

As used herein, "5-Thio-L-Fucose" or "ThioFuc" refers to a fucose derivative whereby the oxygen in the sugar ring is substituted by a sulfur atom. "5-Thio-L-Fucose" means the alpha and/or the beta anomer.

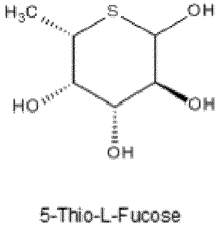

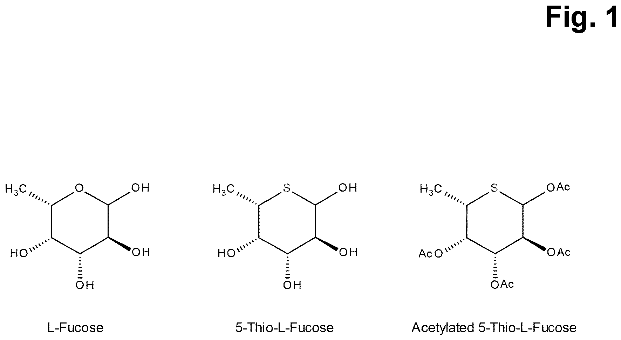

As used herein, "acetylated 5-Thio-L-Fucose" or "AcThioFuc" refers to all forms of acetyl groups on the thiofucose, that means 5-Thio-L-fucose carrying one to four acetyl groups. Figure 1 shows for example peracetylated 5-Thio-L-fucose carrying four acetyl groups.

Derivatives of 5-Thio-L-Fucose are non-, partly or fully acetylated forms of 2-F-5-Thio-L-Fucose, 5-alkynyl-5-thio-L-fucose, 6, 6, 6-Trifluoro-5-thio-L-fucose, 5-Thio-L-Fucose phosphonate.

Non-, partly or fully acetylated forms of 2-F-5-thio-L-Fucose are molecules according to Formula I: 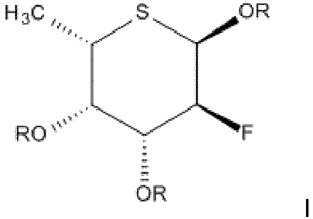 with R being independently of each other H or Acetyl.

Non-, partly or fully acetylated forms of 5-Alkynyl-5-thio-L-fucose are molecules according to Formula II: 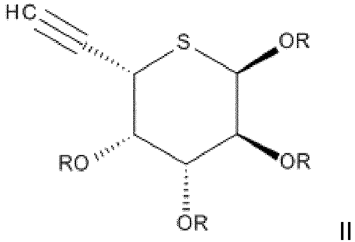 with R being independently of each other H or Acetyl.

Non-, partly or fully acetylated forms of 6, 6, 6-Trifluoro-5-thio-L-fucose are molecules according to Formula III: 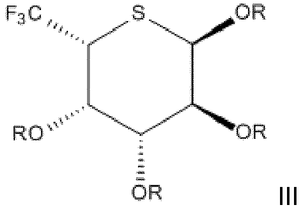 with R being independently of each other H or Acetyl.

Non-, partly or fully acetylated forms of 5-Thio-L-Fucose phosphonate are molecules according to Formula IV: 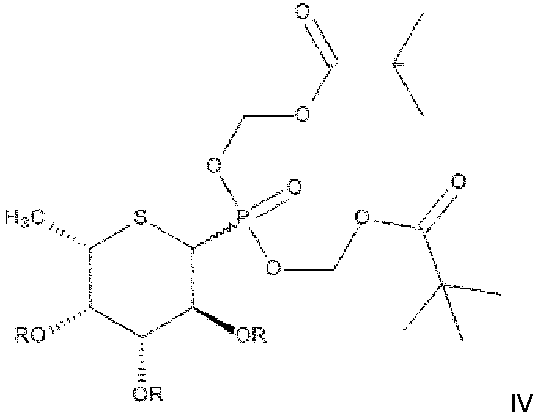 with R being independently of each other H or Acetyl.

The following pictures show the non-acetylated forms. In the acetylated forms, one or more of the OH groups are acetylated.

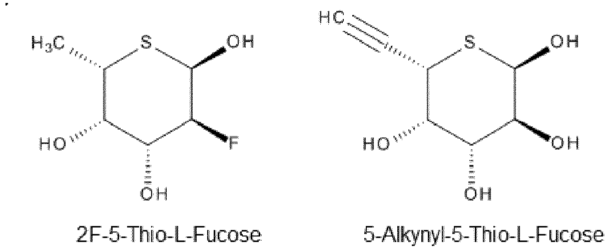

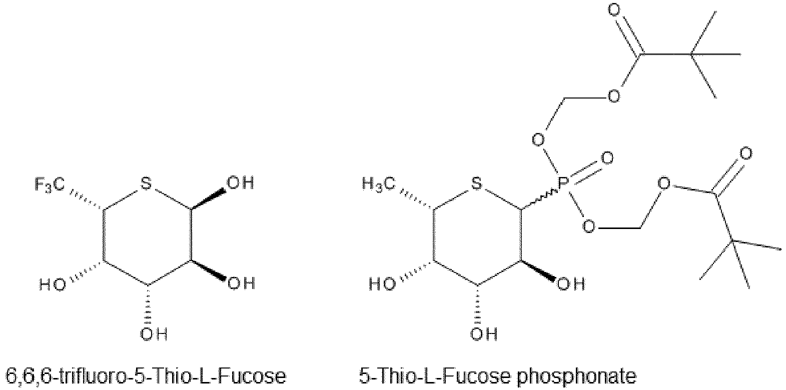

As used herein, "core fucosylation" or "fucosylation" refers to the addition of fucose to N- acetylglucosamine ("GlcNAc") at the reducing terminal of an N-linked glycan.

As used herein, a " N-glycoside- linked sugar chain" or " N-glycoside- linked glycan" is typically bound to asparagine 297 (according to the number of Kabat), although a complex N-glycoside linked sugar chain can also be linked to other asparagine residues. As used herein, the complex N-glycoside-linked sugar chain has a biantennary composite sugar chain, mainly having the following structure: 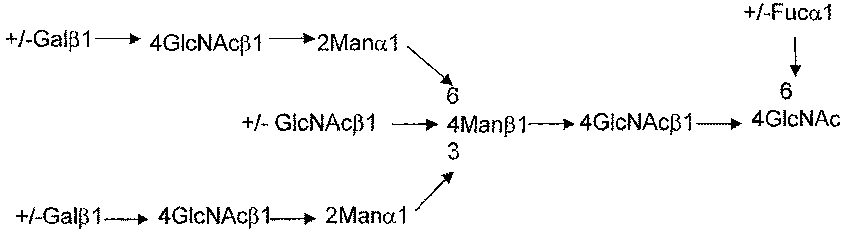 where +/- indicates the sugar molecule can be present or absent, and the numbers indicate the position of linkages between the sugar molecules. In the above structure, the sugar chain terminal which binds to asparagine is called a reducing terminal (at right), and the opposite side is called a non-reducing terminal. Fucose is usually bound to N-acetylglucosamine ("GlcNAc") of the reducing terminal, typically by an α 1,6 bond (the 6-position of GlcNAc is linked to the 1 -position of fucose). "Gal" refers to galactose, and "Man" refers to mannose.

A "complex N-glycoside-linked sugar chain" excludes a high mannose type of sugar chain, in which only mannose is incorporated at the non-reducing terminal of the core structure, but includes
1) a complex type, in which the non-reducing terminal side of the core structure has one or more branches of galactose-N-acetylglucosamine (also referred to as "gal-GlcNAc") and the non-reducing terminal side of Gal-GlcNAc optionally has a sialic acid, bisecting N-acetylglucosamine or the like; or
2) a hybrid type, in which the non- reducing terminal side of the core structure has both branches of the high mannose N- glycoside-linked sugar chain and complex N-glycoside-linked sugar chain.

In some embodiments, the "complex N-glycoside-linked sugar chain" includes a complex type in which the non-reducing terminal side of the core structure has zero, one or more branches of galactose-N-acetylglucosamine (also referred to as "gal-GlcNAc") and the non-reducing terminal side of Gal-GlcNAc optionally further has a structure such as a sialic acid, bisecting N-acetylglucosamine or the like.

The gist of the present invention is to provide reagents and methods to reduce core fucosylation, i.e. fucosylation of the N- glycoside linked sugar chain of an Fc containing protein or antibody. In one embodiment, fucosylation is inhibited and thus reduced or fully suppressed. In another embodiment, the N- glycoside linked sugar chain of an Fc containing protein or antibody is thiofucosylated instead. Both effects can also occur in parallel, either in different antibodies or in one antibody carrying two different glycan structures.

Also provided are antibodies produced by such methods. In other aspects culture media comprising an effective amount of 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate are provided. In the following the derivatives are not specified every time. It is obvious that if 5-Thio-L-fucose is mentioned it also encompasses the above identified derivatives. Preferred is nevertheless 5-Thio-L-fucose and/or partially or fully acetylated 5-Thio-L-fucose.

In some embodiments, fucosylation of complex N-glycoside-linked sugar chains bound to the Fc region (or domain) is fully suppressed or reduced compared to the antibody or other Fc containing protein from the same host cell cultured under the otherwise same culturing conditions but in the absence of 5-Thio-L-fucose. "Under the otherwise same culturing conditions but in the absence of 5-Thio-L-fucose" means typically that the cell culture contains the same ingredients and is performed under the same conditions, but with the difference that no 5-Thio-L-fucose is present.

To suppress or reduce core fucosylation the host cells are cultured in a cell culture medium comprising 5-Thio-L-fucose and/or derivatives thereof.

In a preferred embodiment, the cell culture medium in which the host cell is cultured does not comprise L-Fucose.

The amount of 5-Thio-L-fucose comprised in the liquid cell culture medium in which the host cells are cultured is an amount effective to decrease fucose incorporation. In this context, an "effective amount" refers to an amount of the 5-Thio-L-fucose that is sufficient to decrease fucose incorporation into a complex N-glycoside-linked sugar chain of an antibody or another Fc containing protein by at least 10 percent, at least 20 percent, at least 30 percent, at least 40 percent or at least 60 percent. Typically the amount is between 0.1 µM and 1 mM.

If feed media are added to the cell culture they might comprise higher amount of 5-Thio-L-fucose.

The amount of the 5-Thio-L-fucose that is effective can be determined by standard cell culture methodologies. For example, cell culture assays may be employed to help identify optimal dosing ranges. The precise amount to be employed also depends on the time of administration, the host cell line, the cell density and the like.

The fucosylation and/or thiofucosylation pattern of an antibody may be modulated by altering the concentration of the 5-Thio-L-fucose in the culture medium and/or the duration of exposure to 5-Thio-L-fucose.

The powdered cell culture media of the present invention are preferably produced by mixing all components and milling them. The mixing of the components is known to a person skilled in the art of producing dry powdered cell culture media by milling. Preferably, all components are thoroughly mixed so that all parts of the mixture have nearly the same composition. The higher the uniformity of the composition, the better the quality of the resulting medium with respect to homogenous cell growth.

The milling can be performed with any type of mill suitable for producing powdered cell culture media. Typical examples are ball mills, pin mills, fitz mills or jet mills. Preferred is a pin mill, a fitz mill or a jet mill, very preferred is a pin mill.

A person skilled in the art knows how to run such mills.

A large scale equipment mill with a disc diameter of about 40 cm is e.g. typically run at 1-6500 revolutions per minute in case of a pin mill, preferred are 1-3000 revolutions per minute.

The milling can be done under standard milling conditions resulting in powders with particle sizes between 10 and 300 µm, most preferably between 25 and 100 µm.

Preferably, all components of the mixture which is subjected to milling are dry. This means, if they comprise water, they do only comprise water of crystallization but not more than 10%, preferably not more than 5% most preferred not more than 2% by weight of unbound or uncoordinated water molecules.

In a preferred embodiment, the milling is performed in an inert atmosphere. Preferred inert protective gas is nitrogen.

In another preferred embodiment, all components of the mixture are freezed prior to milling. The freezing of the ingredients prior to the milling can be done by any means that ensures a cooling of the ingredients to a temperature below 0°C and most preferably below - 20°C. In a preferred embodiment the freezing is done with liquid nitrogen. This means the ingredients are treated with liquid nitrogen, for example by pouring liquid nitrogen into the container in which the ingredients are stored prior to introduction into the mill. In a preferred embodiment, the container is a feeder. If the container is a feeder the liquid nitrogen is preferably introduced at the side or close to the side of the feeder at which the ingredients are introduced.

Typically the ingredients are treated with the liquid nitrogen over 2 to 20 seconds.

Preferably the cooling of the ingredients is done in a way that all ingredients that enter into the mill are at a temperature below 0°C, most preferred below - 20°C.

In a preferred embodiment, all ingredients are put in a container from which the mixture is transferred in a feeder, most preferred in a metering screw feeder. In the feeder the ingredients are sometimes further mixed - depending on the type of feeder - and additionally cooled. The frozen mixture is then transferred from the feeder to the mill so that the mixture which is milled in the mill preferably still has a temperature below 0°C, more preferred below - 20 °C.

Typically the blending time, that means the residence time of the mixture of ingredients in the feeder is more than one minute, preferably between 15 and 60 minutes.

A metering screw feeder, also called dosage snail, is typically run at a speed of 10 to 200 revolutions per minute, preferably it is run at 40 to 60 revolutions per minute.

Typically, the temperature of the mill is kept between -50 and +30°C. In a preferred embodiment, the temperature is kept around 10°C.

The oxygen level during milling preferably is below 10% (v/v).

The process can be run e.g. batch-wise or continuously. In a preferred embodiment the process according to the present invention is done continuously by, over a certain time, permanently filling the mixture of ingredients into a feeder for cooling and permanently filling cooled mixture from the feeder into the mill.

After milling, the resulting dry powder medium might be further compacted to enlarge the size of the particles, e.g. by dry compaction in a roll press.

For use of the dry powdered media resulting, e.g. resulting from milling or wet or dry compaction, a solvent, preferably water (most particularly distilled and/or deionized water or purified water or water for injection) or an aqueous buffer is added to the media and the components are mixed until the medium is totally dissolved in the solvent and the ready to use liquid medium is generated.

The solvent may also comprise saline, soluble acid or base ions providing a suitable pH range (typically in the range between pH 1.0 and pH 10.0), stabilizers, surfactants, preservatives, and alcohols or other polar organic solvents.

It is also possible to add further substances like buffer substances for adjustment of the pH, fetal calf serum, sugars etc. to the mixture of the cell culture medium and the solvent. The resulting liquid cell culture medium is then contacted with the cells to be grown or maintained.

The dry powder or granulated medium according to the present invention typically comprises 5-Thio-L-fucose in an amount so that after dissolution the resulting liquid medium comprises an effective amount of 5-Thio-L-fucose, typically between 10 nmol/l and 100 mmol/l, preferably between 0.1 µmol/l and 10 mmol/l. The dry powder or granulated medium preferably does not contain any L-fucose.

The present invention is further directed to a process for culturing cells by
a) providing a bioreactor
b) mixing the cells to be cultured with a cell culture medium generated by dissolving a dry powder medium according to the present invention
c) incubating the mixture of step b)

In one embodiment the bioreactor is a perfusion bioreactor.

A bioreactor is any vessel, flask or tank in which cells can be cultured. Incubation is typically done under suitable conditions like suitable temperature etc. A person skilled in the art is aware of suitable incubation conditions for supporting or maintaining the growth/culturing of cells such as suitable temperature, pH, osmolality, aeration, agitation, etc. and a bioreactor which limits or ideally avoids contamination with foreign microorganisms from the environment.

A perfusion bioreactor is a bioreactor in which perfusion cell culture can be performed. It comprises the bioreactor vessel which is typically closed during cell culture, a stirrer in the vessel, a line for introducing fresh medium, a harvest line for removing the harvest stream comprising cells, liquid medium and target product from the bioreactor and a cell retention device in the harvest line that retains the cells while the liquid part of the harvest can be collected. A review about perfusion cell culture providing details about favorable set ups can be found in "Perfusion mammalian cell culture for recombinant protein manufacturing - A critical review" Jean-Marc Bielser et al., Biotechnology Advances 36 (2018) 1328-1340.

The present invention is also directed to a process for culturing cells in a bioreactor by
- Filling into a bioreactor cells and an aqueous cell culture medium
- Incubating the cells in the bioreactor
- Continuously over whole time of the incubation of the cells in the bioreactor or once or several times within said incubation time adding a cell culture medium to the bioreactor
whereby the medium preferably has a pH of less than pH 8.5 and comprises at least 5-Thio-L-fucose and/or a derivative thereof as defined above.

In a preferred embodiment the medium is a feed medium.

Typically a feed medium comprises between 50 and 300 g/l of solid ingredients that are dissolved in the solvent.

In another embodiment the medium is a perfusion medium.

In one embodiment, in the process of the present invention the feed medium that is added during the incubation either continuously or once or several times within said time to the bioreactor has a differing composition.

In another, typically preferred embodiment, in the process of the present invention the feed medium that is added during the incubation either continuously or once or several times within said time to the bioreactor always has the same composition.

The antibodies and antibody derivatives produced by the methods of the present invention can be isolated from the cell culture and purified using, for example, gel electrophoresis, filtration, dialysis, and chromatography like e.g. affinity chromatography and/or ion exchange chromatography.

In some embodiments, the antibodies or antibody derivatives produced by the methods of the present invention have higher effector function (e.g. ADCC activity) than the antibodies or antibody derivatives produced in the absence of 5-Thio-L-fucose. ADCC activity may be measured using assays known in the art and in exemplary embodiments increases by at least 10 percent, 20 percent, 30 percent, 40 percent, 50 percent, 60 percent, 70 percent, 80 percent, 90 percent, 2-fold, 3-fold, 4-fold, 5-fold, 6-fold, 7-fold, 8-fold, 9-fold, 10- fold, 15-fold or 20-fold, as compared to the core fucosylated parent antibody.

5-Thio-L-fucose and/or derivatives thereof are especially suitable to modify core fucosylation as they typically do not have significant impact on the cellular performance (VCD and titer). Glycosylation patterns of antibodies produced by the methods of the present invention show that sometimes core fucosylation is reduced whereby less overall core fucosylation occurs and sometimes core fucosylation is reduced due to its replacement by thiofucosylation. Sometimes both effects can be observed simultaneously. Typically the fucosylation level can be decreased by using increasing amounts of 5-Thio-L-Fucose and in parallel the incorporation of 5-Thio-L-Fucose is increased by using increasing amounts of 5-Thio-L-Fucose, whereby the effect is not completely antiparallel so that afucosylation and also thiofucosylation can be modified by careful selection of the concentration of 5-Thio-L-Fucose that is added to the cell culture.

5-Thio-L-Fucose shows a higher efficiency compared to the known inhibitor 2F-Peracetylfucose. As can be seen in Example 4, production of an antibody with 5-Thio-L-Fucose treatment showed 64%, 68% and 67% reduction of the fucosylation compared to the control on day 7,10 and 12, respectively. In comparison, addition of the same concentration of 2F-Peracetylfucose, a known fucose-analogue, was only able to achieve a reduction of 11 %, 50 % and 58 % on day 7, 10 and 12, respectively.

It could further be shown that antibodies produced with the methods of the present invention with media comprising 5-Thio-L-Fucose show a higher binding affinity to their receptor.

The present invention is further illustrated by the following figures and examples, however, without being restricted thereto.

### Examples

The following examples represent practical applications of the invention.

### Example 1: Expression of recombinant proteins in presence of 5-Thio-L-Fucose.

A common fed-batch process was used to determine the effect of 5-Thio-L-Fucose (Fig 1) on the glycosylation profile of recombinant proteins. Four different clones of Chinese hamster ovary (CHO) cells expressing three different monoclonal antibodies (mAb) and a fusion protein were tested. Cells were cultivated in spin tubes between 14 and 20 days at 37 °C, 5% CO₂, and 80% humidity. As starting cell density, 3 × 10⁵ cells/mL in 30 mL Cellvento^{®}4CHO media (pH 7.0 ± 0.1) was used for done 1 and 2 producing mAb1 and mAb2, respectively (Cellvento^{®} Platform). Cells were cultivated at an agitation rate of 320 rpm and were fed on day 3, 5, 10, 12 and 14 with 3 % volume of Cellvento^{®} 4Feed (v/v) and on day 7 with 6 % (v/v) of the feed. Clone 3 and 4 producing mAb3 and a fusion protein were inoculated at the same cell density in 30 mL Ex-Cell^{®} Advanced Media (pH 7.2 ± 0.1) while agitation was set to 230 rpm. Clones 3 and 4 were fed with 5% Ex-Cell^{®} Advanced Feed (v/v) on day 3, 5, 7, 10, 12, 14 and 17. Feeds were supplemented with either 400 µM 5-Thio-L-Fucose, 400 µM acetylated 5-Thio-L-Fucose, 400 µM acetylated 2F-Fucose as positive control or the respective amount of DMSO (1.17%) used as solvent for both acetylated fucose analogues. The pH of all Cellvento^{®} feeds was adjusted to 7.0 ± 0.1 and Ex-Cell^{®} Feeds were adjusted to 8.5 ± 0.1.

The glucose level was maintained above 4 g/L by adding a specific amount of a 400 g/L glucose stock solution on demand to up to 6 g/L during the week and up to 13 g/L over the weekend. Glucose and titer were detected in the supernatant with the bioprocess analyzer CEDEX Bio HT (Roche, Mannheim, Germany) based on spectrophotometric and turbidometric methods. The viable cell density (VCD) and viability were evaluated with a Vi-CELL^{™} XR 2.04 cell counter (Beckman Coulter, Fullerton, CA, USA).

In general, 5-Thio-L-Fucose treatment was compared to the control condition without any supplements. Acetylated 5-Thio-L-Fucose was compared to the known positive control 2F-Peracetylfucose and the DMSO control used to solve both acetylated fucose analogues.

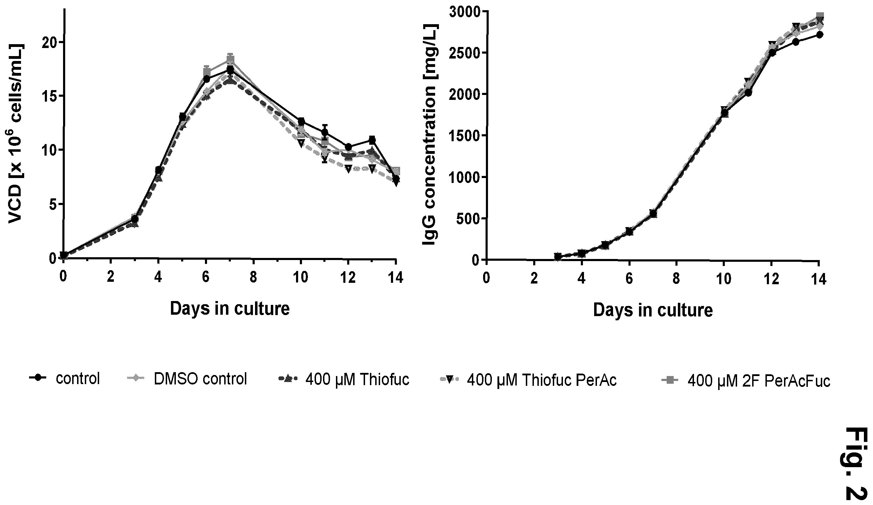

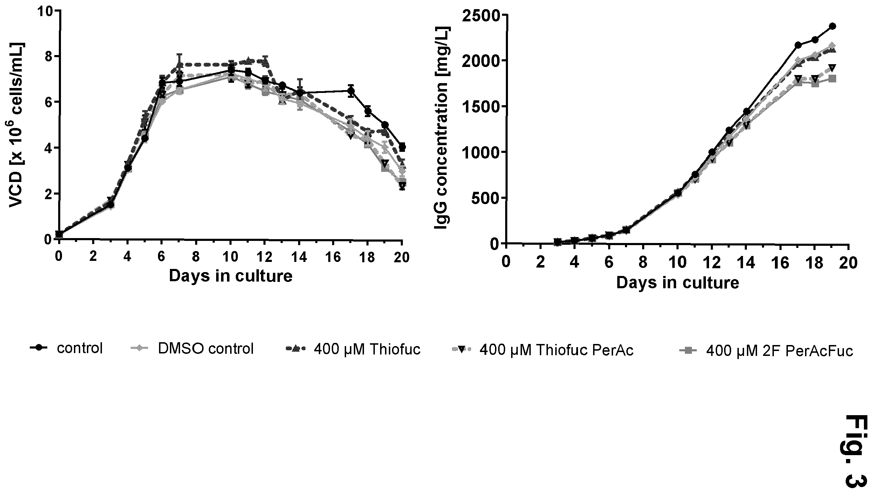

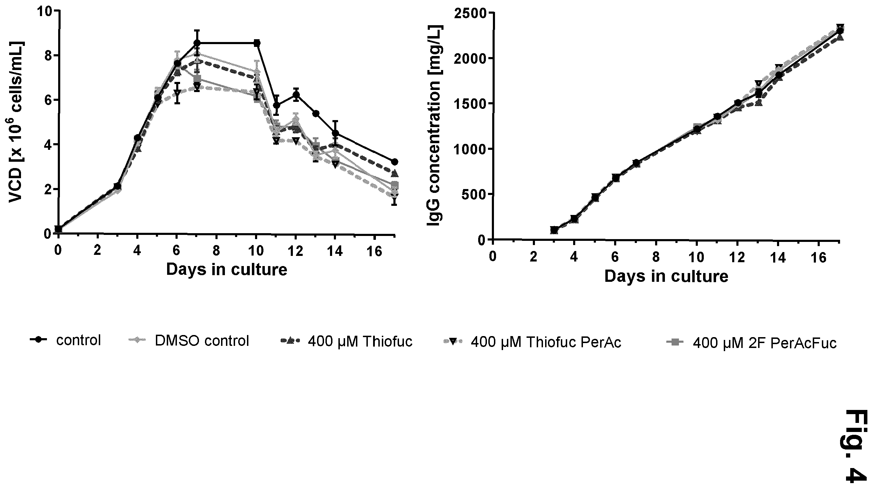

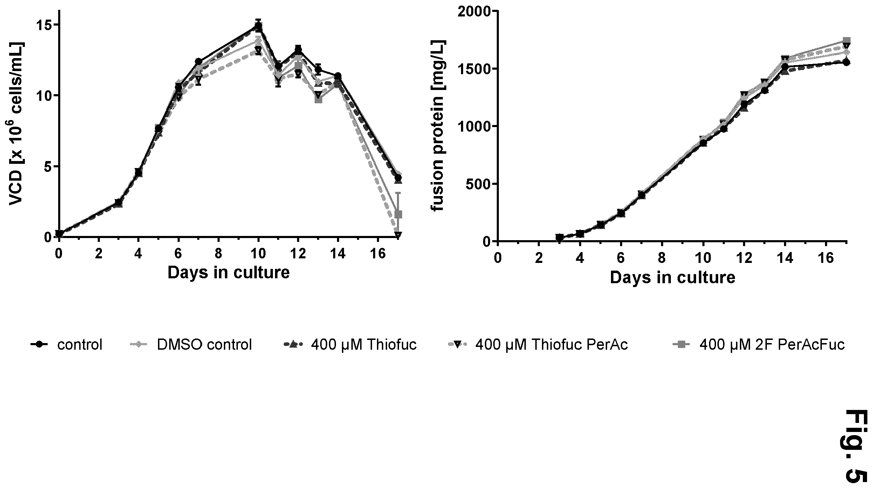

As shown in Figures 2 to 5, within four biological replicates, 5-Thio-L-Fucose treatment with or without acetylation showed no significant impact on the cellular performance (VCD and titer) of these four investigated CHO cell lines when compared to two control conditions and a positive control. Slight changes were detected in both platforms for mAb2 and mAb3. Production of mAb2 resulted in a slightly reduced titer after day 12 in all conditions compared to the non-supplemented control. On day 17, the titer of 2.18 g/L in the control was reduced to 2.02 g/L and 1.99 g/L with 5-Thio-L-Fucose treatment and the DMSO control, respectively. Further reduction to 1.82 g/L was detected with acetylated 5-Thio-L-Fucose to a similar level as the positive control 2F-Peracetylfucose (1.78 g/L). Since both peracetylated fucose analogues were solved in DMSO the reduced titer is partly due to the solvent.

For production of mAb3, a reduced VCD of about 13 % and 24 % was detected for 5-Thio-L-Fucose and acetylated 5-Thio-L-Fucose by calculating the area under the curve until day 17 (compared to the control). The DMSO control showed a similar reduction of 13 %, indicating that the solvent of acetylated 5-Thio-L-Fucose most probably led to the further reduction of the VCD. Since the reduced VCD did not affect the titer (2.25 g/L to 2.33 g/L) it is sought to be within the biological variability of the experiment.

### Example 2: 5-Thio-L-Fucose is incorporated into the glycan structure

The glycosylation profile of the recombinant proteins produced in the control fed-batch was compared to the profile obtained in fed-batch processes supplemented with the fucose analogues. The antibody was purified from the cell culture supernatant using protein A PhyTips^{®} (PhyNexus Inc, San Jose, CA).

Glycosylation patterns were analyzed by ultra-performance liquid chromatography coupled to a mass spectrometer (UPLC-MS). The purified antibody was denaturated and the glycans were released from the antibody by digestion with peptide N-glycosidase F (PNGase F) followed by labeling the glycans with the GlycoWorks^{™} RapiFluor-MS^{™} N-Glycan Kit according to the manufacturer protocol. For the analysis, an UPLC glycan column (2.1 × 150 mm) that separated the glycans according to their hydrophobicity was used. The chromatography was performed at 45°C using 50 mM ammonium formate (A), pH 4.4 solution and an acetonitrile (B) gradient. In detail, at the beginning the ratio of A:B was 20:80 (0 min) and changed to 27:73 at 3 min and 37: 63 at 35 min at a flow rate of 0.5 ml/min. From 36.5 min to 39.5 min only A was used as mobile phase and the flow rate was reduced to 0.2 ml/min. Subsequently, the ratio was changed to 20 % A: 80 % B and the flowrate was increased to 0.5 ml/min at 43.1 min until the end of the process at 55 min. The detection was performed by a fluorescence detector with an excitation wavelength of 265 nm and an emission wavelength of 425 nm. The identification of the glycan structures was performed by MS using positive electro spray ionization (ESI), which was calibrated every 30 s with a standard.

Glycans containing the unnatural 5-Thio-L-fucose were identified through a retention time shift, a mass shift and the fragmentation pattern. The mass shift of about +16 Da is caused by the exchange of the ring oxygen in fucose (164.0684 g/mol) with sulfur in the Thio-L-Fucose derivative leading to a theoretical mass of 180.0455 g/mol for the 5-Thio-L-Fucose derivative. This mass is similar to the mass of galactose with 180.0633 g/mol but can be distinguished by higher resolution mass spectrometers (mass difference of 115 ppm).

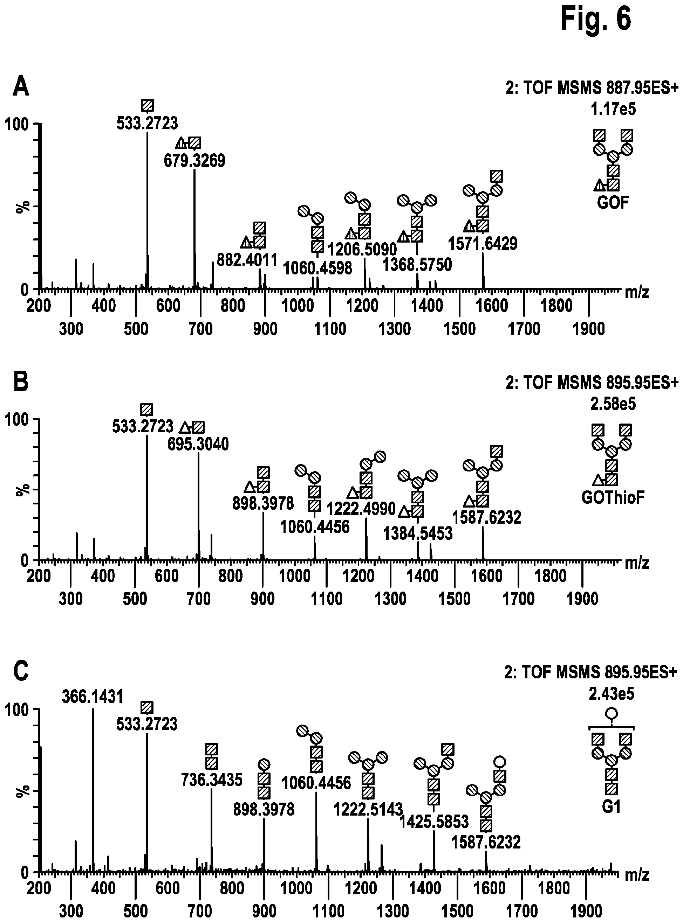

Furthermore, fragment spectra were obtained using LC-MS/MS. The fragmentation pattern of glycans containing thiofucose is similar to the respective fucosylated glycan fragmentation pattern but differs for every glycan fragment containing fucose. For example, the Rapifluor-GlcNAc-Fuc fragment presents a theoretical mass of 679.3304 g/mol whereas the Rapifluor GlcNAc-thioFuc presents a theoretical mass of 695.3076 g/mol. The difference allows a definitive identification of the sugar structures (Fig 6, **A** fucosylated G0F, **B** thiofucosylated G0ThioF and **C** G1 fragmentation pattern)

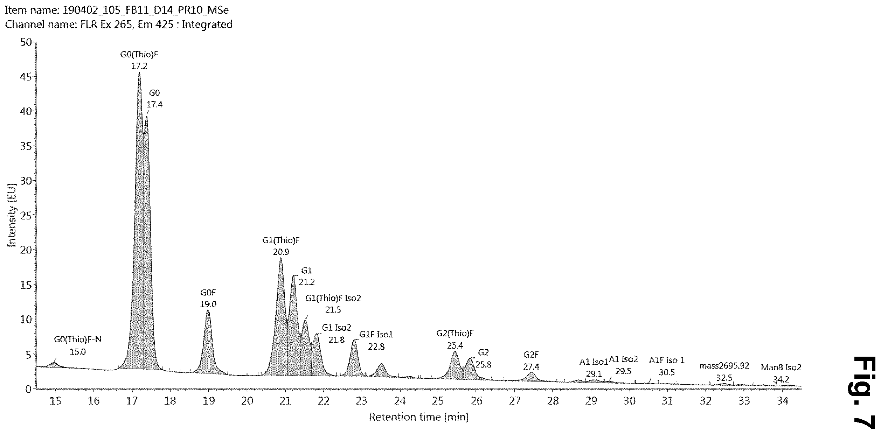

Additionally, the retention time of thiofucosylated glycans can be distinguished from fucosylated glycans. In Fig 7, an exemplarily shift in retention time of the fluorescence peaks is shown for a mAb treated with 800 µM 5-Thio-L-Fucose. The thiofucosylated G0F peak was detected at 17.19 min whereas elution of the G0 peak occurs at 17.38 min. Separation of thiofucosylated G1F isoforms peaks can be observed at 20.88 and 21.52 min compared to the retention time of both fucosylated G1F isoforms at 22.81 and 23.51 min. Overall, this data confirms the replacement of core-fucosylation by 5-Thio-L-Fucose in the glycan structure.

Fig 7 shows the fluorescence signal obtained after separation of the released, labelled glycans using UPLC, showing a changed retention time of the peaks corresponding to thiofucosylated glycans compared to fucosylated glycans.

### Example 3: 5-Thio-L-Fucose can reduce fucosylation through incorporation of 5-Thio-L-Fucose.

A common fed-batch process was used to determine the effect of 5-Thio-L-Fucose on the glycosylation profile. Four different clones of Chinese hamster ovary (CHO) cells expressing three different monoclonal antibodies (mAb) and a fusion protein were tested. Cells were cultivated in spin tubes between 14 and 20 days at 37 °C, 5% CO₂, and 80% humidity. As starting cell density, 3 × 10⁵ cells/mL in 30 mL Cellvento^{®}4CHO media (pH 7.0 ± 0.1) was used for clone 1 and 2 producing mAb1 and mAb2, respectively (Cellvento^{®} Platform). Cells were cultivated at an agitation of 320 rpm and were fed on day 3, 5, 10, 12 and 14 with 3 % volume of Cellvento^{®} 4Feed (v/v) and on day 7 with 6 % (v/v) of the feed. Clone 3 and 4 producing mAb3 and a fusion protein were inoculated at the same cell density in 30 mL Ex-Cell^{®} Advanced Media (pH 7.2 ± 0.1) while agitation was set to 230 rpm (Ex-Cell^{®} Platform). Clones 3 and 4 were fed with 5 % Ex-Cell^{®} Advanced Feed (v/v) on day 3, 5, 7, 10, 12, 14 and 17. Feeds were supplemented with either 400 µM 5-Thio-L-Fucose, 400 µM acetylated 5-Thio-L-Fucose, 400 µM acetylated 2F-Fucose as positive control or the respective amount of DMSO (1.17 %) used as solvent for both acetylated fucose analogues. The pH of all Cellvento^{®} feeds was adjusted to 7.0 ± 0.1 and Ex-Cell^{®} Feeds were adjusted to 8.5 ± 0.1. The glucose level was maintained above 4 g/L by adding a specific amount of a 400 g/L glucose stock solution on demand to up to 6 g/L during the week and up to 13 g/L over the weekend. Glucose and titer were detected in the supernatant with the bioprocess analyzer CEDEX Bio HT (Roche, Mannheim, Germany).

To investigate the glycosylation profile during the fed-batch experiment samples were centrifugated and antibodies and Fc-fusion proteins were purified from the cell culture supernatant using protein A PhyTips^{®} (PhyNexus Inc, San Jose, CA). Glycosylation patterns were analyzed as described by ultra-performance liquid chromatography coupled to a mass spectrometer (UPLC-MS) using the GlycoWorks^{™} RapiFluor-MS^{™} N-Glycan Kit.

In general, 5-Thio-L-Fucose treatment with or without acetylation was compared to the control condition without any supplements, to the known positive control 2F-Peracetylfucose and the DMSO control used to solve both acetylated fucose analogues. Glycosylation profile on day 12 was visualized since harvest of recombinant produced proteins on day 12 is common. Quantification of the glycans was obtained using the relative peak area of the fluorescence signal. Almost all peaks were assigned to glycan structures according to their mass. Unknown glycan structures and signals without mass detection were summarized as unknown species (not shown).

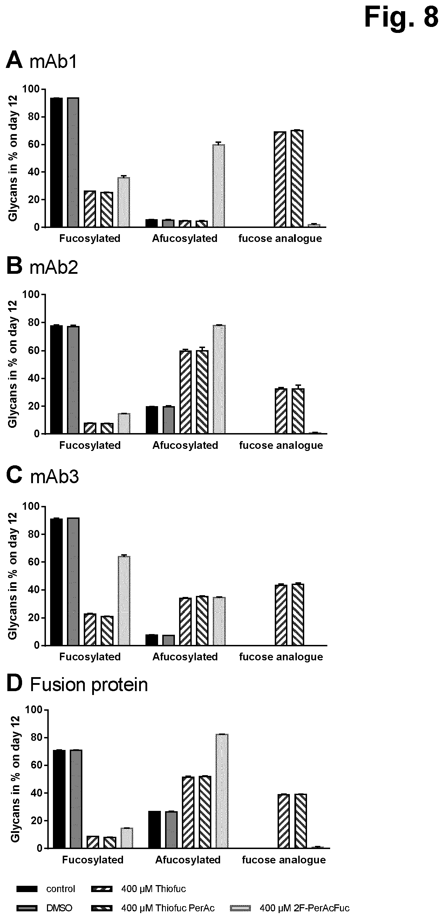

Four different clones of Chinese hamster ovary (CHO) cells expressing three different monoclonal antibodies (mAb) and a fusion protein were tested. Core-fucosylation was reduced efficiently through 5-Thio-L-Fucose treatment in all tested cell lines producing different recombinant proteins. Reduced fucosylation of mAb1 produced in clone 1 by 5-Thio-L-Fucose was achieved through high incorporation of the fucose-analogue. Addition of 400 µM 5-Thio-L-Fucose with or without acetylation resulted in a reduction of core-fucosylated glycans to about 26 % on day 12 compared to 94 % core-fucosylated glycans in the control (Fig 8 A). The addition of the same concentration of acetylated 2F-Fucose, a known fucose-analogue reducing fucosylation, was less efficient leading to 36 % core-fucosylation on day 12. In contrast to acetylated 2F-Fucose, the reduction of the native fucosylation by 5-Thio-L-Fucose was achieved through high incorporation of 5-Thio-L-Fucose to about 70 % of the glycans, regardless of the acetylation. Afucosylation was only increased by acetylated 2F-Fucose to 60 % on day 12 compared to 5 % for the control and 5-Thio-L-Fucose. Production of mAb2 resulted in 8 % core-fucosylation on day 12 after 5-Thio-L-Fucose treatment with or without acetylation compared to 77 % fucosylation in the control (Fig 8 B). In contrast to mAb1 for which the afucosylation level was not changed in response to 5-Thio-L-Fucose treatment, the reduction in fucosylation of mAb2 was achieved by 32 % incorporation of 5-Thio-L-Fucose and 60 % afucosylation (compared to 20% in the control condition). The known positive control achieved a 14 % fucosylation level on day 12 by increasing the afucosylation to 78 %.

The same amount of afucosylation compared to the known fucose-analogue acetylated 2F-Fucose was only achieved by clone 3 expressing mAb3 leading to about 34 % afucosylation on day 12 (Fig 8 C). 5-Thio-L-Fucose was 43 % incorporated leading to a lower fucosylation level of 21 % compared to 90 % in the control and 65 % in the positive control on day 12. A similar glycosylation pattern as for mAb2 was observed for the fusion protein produced in clone 4 on day 12 (Fig 8 D). Compared to the control core-fucosylation was reduced about 63 % via 5-Thio-L-Fucose treatment with or without acetylation. For both, incorporation of 5-Thio-L-Fucose was observed in 39 % of the glycans and 52 % were afucosylated. The positive control reached 82 % afucosylation without detectable incorporation of the fucose analogue.

Overall, increasing amounts of incorporated 5-Thio-L-Fucose were detected on day 12 for mAb2, the fusion protein, mAb3 and mAb1 with about 32 %, 39 %, 43 %, and 70 %, respectively.

Increasing amounts of afucosylation were detected for mAb1, mAb3, the fusion protein and mAb2 with about 5 %, 34 %, 52 % and 60 %.

Fig 8 shows the impact of 5-Thio-L-Fucose on fucosylation profile using CHO cell lines producing (A) mAb1, (B) mAb2, (C) mAb3 and (D) a fusion protein. Data presents mean values ± SEM of two biological replicates on day 12.

### Example 4: The addition of 5-Thio-L-Fucose to the process leads to a quicker reduction of fucosylation compared to the addition of 2F-Peracetylfucose.

To identify the time required by 5-Thio-L-Fucose supplementation to influence the glycosylation profile of recombinant produced proteins, samples taken at different time points during the fed-batch were investigated. Samples were collected on day 5, 7, 10, 12 and 14 with a cell viability above 60%. After centrifugation, the antibodies and Fc-fusion proteins were purified from the cell culture supernatant using protein A PhyTips^{®} (PhyNexus Inc, San Jose, CA). Glycosylation patterns were analyzed as described by ultra-performance liquid chromatography coupled to a mass spectrometer (UPLC-MS) using the GlycoWorks^{™} RapiFluor-MS^{™} N-Glycan Kit.

Early investigation of the glycan profile during a fed-batch experiment indicated a high efficiency of 5-Thio-L-Fucose. Production of mAb1 with 5-Thio-L-Fucose treatment showed 64%, 68% and 67% reduction of the fucosylation compared to the control on day 7,10 and 12, respectively. In comparison, addition of the same concentration of 2F-Peracetylfucose, a known fucose-analogue, was only able to achieve a reduction of 11 %, 50 % and 58 % on day 7, 10 and 12, respectively. This indicates a higher efficiency of 5-Thio-L-Fucose compared to the known inhibitor 2F-Peracetylfucose.

To detect the impact of 5-Thio-L-Fucose earlier in the fed-batch, the glycosylation profile of mAb3 was additionally investigated on day 5. The detected fucosylation level obtained with the 400 µM 5-Thio-L-Fucose treatment on day 5 was already at a low level of about 48 % whereas 400 µM acetylated 2F-Fucose resulted in about 84 % fucosylation on day 5, a similar level as detected in the control with 87 % fucosylation. On day 7, 10, 12 and 14 fucosylation level of the control was constant between 87 % and 90 %, whereby 5-Thio-L-Fucose treatment reduced the core-fucosylation to about 34 %, 25 %, 21 %, and 18 %, respectively. In comparison, the known inhibitor acetylated 2F-Fucose showed only a reduced fucosylation level to about 78 %, 72 %, 65 %, and 57 % on day 7, 10, 12 and 14, respectively. Therefore, 5-Thio-L-Fucose might be favorable for an application with a short cultivation time like a batch or perfusion process as well. In addition, reduced amounts or different feeding regimes may be applied to reduce the required amount of fucose derivative added through the process or to titrate the fucosylation level.

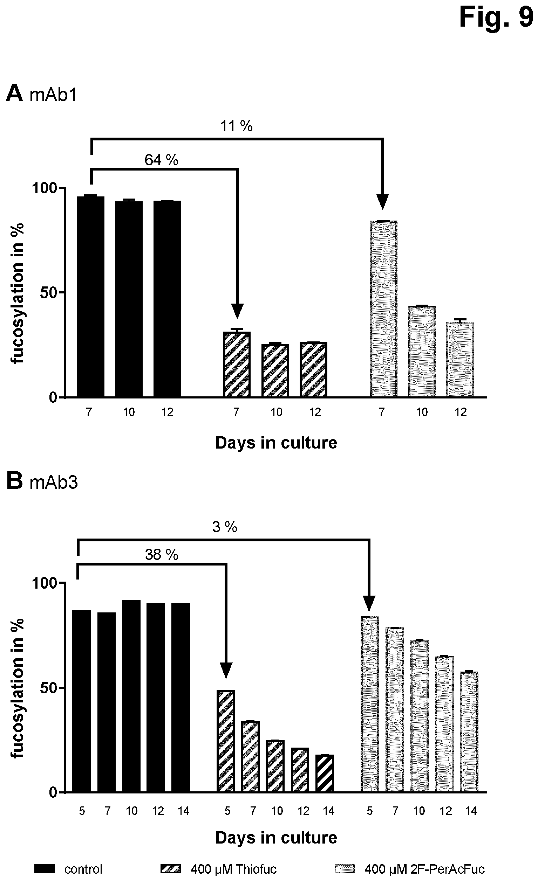

Fig 9 shows the accelerated effect of 5-Thio-L-Fucose during fed-batch experiments producing (A) mAb1 or (B) mAb3. Data represents mean values ± SEM of two biological replicates.

### Example 5: Increasing concentrations of 5-Thio-L-Fucose lead to higher incorporation.

The ability to achieve different fucosylation levels through 5-Thio-L-Fucose addition during a fed-batch was investigated with two platforms and two different CHO clones producing either mAb1 or mAb3.

Cells were cultivated in spin tubes between 14 and 20 days at 37 °C, 5% CO₂, and 80% humidity. As starting cell density, 3 × 10⁵ cells/mL in 30 mL Cellvento^{®} 4CHO media (pH 7.0 ± 0.1) was used for clone 1 producing mAb1 (Cellvento^{®} Platform). Cells were cultivated at an agitation of 320 rpm and were fed on day 3, 5, 10, 12 and 14 with 3 % volume of Cellvento^{®} 4Feed (v/v) and on day 7 with 6 % (v/v) of the feed. Clone 3 producing mAb3 was inoculated at the same cell density in 30 mL Ex-Cell^{®} Advanced Media (pH 7.2 ± 0.1) while agitation was set to 230 rpm (Ex-Cell^{®} Platform). Clones 3 was fed with 5% Ex-Cell^{®} Advanced Feed (v/v) on day 3, 5, 7, 10, 12, 14 and 17.

Feeds were supplemented with increasing concentrations of 5-Thio-L-Fucose, acetylated 5-Thio-L-Fucose, or the respective amount of DMSO (1.17%) used as solvent for acetylated 5-Thio-L-Fucose. The pH of all Cellvento^{®} feeds was adjusted to 7.0 ± 0.1 and Ex-Cell^{®} Feeds were adjusted to 8.5 ± 0.1. The glucose level was maintained above 4 g/L by adding a specific amount of a 400 g/L glucose stock solution on demand to up to 6 g/L during the week and up to 13 g/L over the weekend. Glucose and titer were detected in the supernatant with the bioprocess analyzer CEDEX Bio HT (Roche, Mannheim, Germany).

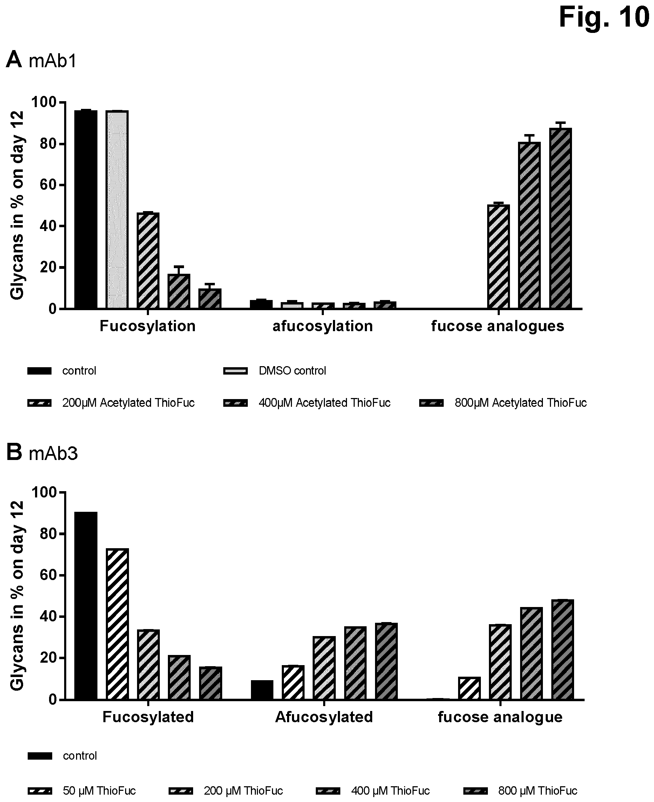

Dose response with clone 1 producing mAb1 was performed with 200, 400 and 800 µM acetylated 5-Thio-L-Fucose (Fig 10 A). Glycosylation profile was investigated on day 7, 10 and 12. Since only minor changes were detected over time, glycan structure of the produced IgG are presented as mean values of all three days. The data indicated increasing incorporation of the unnatural fucose-analogue from 50 % to 81 % and 87 % by adding 200 µM, 400 µM and 800 µM acetylated 5-Thio-L-Fucose, respectively. Thereby, fucosylation level was reduced to 46 %, 16 % and 9 % compared to 96 % fucosylation in both control conditions (including 1.17% DMSO as solvent of acetylated 5-thio-L-Fucose).

Furthermore, the impact of increasing concentrations of 5-Thio-L-Fucose was investigated on clone 3 producing mAb3. Concentrations from 50 µM to 800 µM 5-Thio-L-Fucose yielded an increasing amount of incorporated 5-Thio-L-Fucose as well as an increasing amount of afucosylation. The highest applied concentration of 800 µM 5-Thio-L-Fucose resulted in about 36 % afucosylation and about 48 % incorporated fucose analogue leading to about 15 % remaining native fucosylated glycans on day 12. Consequently, the addition of 800 µM 5-Thio-L-Fucose was able to reduce fucosylation about 75 % on day 12 when compared to the control. Gradual reduction of the concentration to 400 µM, 200 µM and 50 µM 5-Thio-L-Fucose resulted in reduced incorporation of the fucose analogue on day 12 of about 44 %, 36 % and 11 %, respectively. This data indicates that the amount of incorporated 5-Thio-L-Fucose can be targeted by a careful selection of the concentration.

Fig 10 shows the dose response of 5-Thio-L-Fucose on glycosylation profile. (A) Impact of acetylated 5-Thio-L-fucose on CHO cell line producing mAb1. Data represents mean values ± SEM of day 7, 10 and 12. (B) Impact of 5-Thio-L-Fucose on CHO cell line producing mAb3. Data represents mean values ± SEM of two biological replicates on day 12.

### Example 6: Impact of 5-Thio-L-Fucose treatment on FcγRIIIa binding

The aim of this part is to investigate whether antibodies produced in the thiofucose containing process present a higher binding affinity to FcyRIIIa and thus a potentially increased ADCC.

Indeed, increased binding of afucosylated antibodies to FcyRIIIa and thereby enhanced ADCC activity is described in the literature. Two isoforms of FcγRIIIa with either a phenylalanine (F158) or valine (V158) residue in position 158 are known. Later isoform is reported to have a higher affinity to IgG1 compared to the F isoform and was used for these studies.

Binding of samples after 5-ThioFuc treatment was investigated by surface plasmon resonance technology using a Biacore T200 system (GE Healthcare, Uppsala, Sweden). The analysis temperature was set to 25°C and sample compartment temperature to 15°C. Using the His capture Kit from GE Healthcare, a series S Sensor Chip CM5 was used to couple anti-His antibody according to the manufacturer's instructions in the active and the reference flow cell. Immobilization levels were in a range of 12185 ± 436 resonance units (RU; 1 RU ≈ 1 pg/mm²) for all experiments. The anti-His antibody was further used to capture His-tagged FcγRIIIa V176 (CD8-H52H4) from AcroBiosystems (Newark, DE, USA). The receptor was expressed in HEK293 cells and 0.011 µg/mL were injected in the active flow cell for 60 s at a flow rate of 10 µL/min, resulting in capture levels of 4.5 ± 0.2 RU. Subsequently, mAb3 was injected for 150 s at a flow rate of 30 µL/min with five increasing concentrations in a range from 1.9, 5.6, 16.7, 50, 150 and 450 nM over both, the reference and the active flow cell. Binding was recorded continuously, while unspecific binding and injection artefacts were subtracted automatically. Single-cycle kinetics were used with a dissociation time of 600 s at a flow rate of 30 µL/min. Data collection rate was performed at 10 Hz. Following each experiment, both flow cells were regenerated for 30 s with 10 mM glycine-HCl pH 1.5 according to kit instructions. Data of three technical and two biological replicates were fitted with a 1:1 binding model. The global dissociation constant KD was determined from the ratio of the kinetic rate constants for dissociation (kd) and association (ka) values. Data are presented in Table 1 as fold change compared to the control.

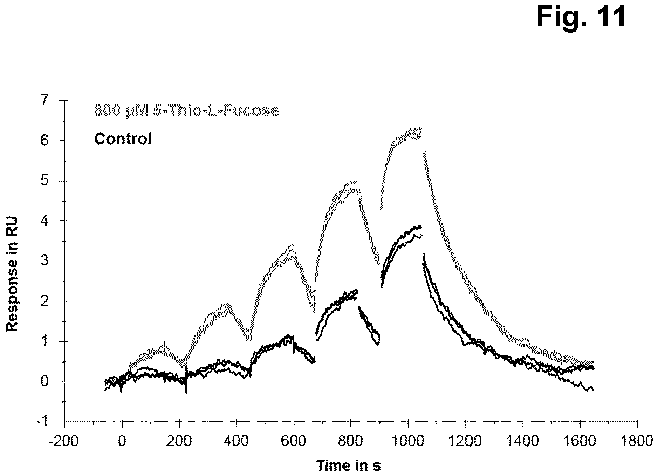

Cells treated with increasing concentrations of 5-Thio-L-Fucose produced antibodies with increasing affinity to the FcγRIIIa. Samples from day 5 treated with either 50 µM 5-Thio-L-Fucose or 400 µM 2F-PerAcFuc achieved a 1.2 and 1.3-fold enhanced binding compared to the control. Samples treated with 200, 400 and 800 µM 5-Thio-L-Fucose showed on day 5 increased affinity compared to the control by 2.0, 2.9 and 2.6-fold, respectively. At later time points increased affinity to FcγRIIIa was observed for all treated samples. On day 12 increased affinity of 2.0, 3.1, 3.8 and 4.8-fold was detected by 50, 200, 400 and 800 µM 5-Thio-L-Fucose treatment, respectively. The sensorgram resulting from injection of the same concentration series of control and treated antibodies (Fig 11) illustrates the higher affinity through a higher response of the antibody treated with 5-Thio-L-Fucose compared to the control on day 12. Additionally, treatment with 400 µM 5-Thio-L-Fucose showed a slightly higher binding affinity compared to 400 µM 2F-PerAcFuc, a known inhibitor, leading to 3.6-fold increased affinity. On day 14 treated samples showed similar KD values compared to day 12, due to similar glycosylation pattern. Enhanced binding by 1.7, 3.6, 4.1 and 4.5-fold through 5-Thio-L-Fucose treatment was detected at 50, 200, 400 and 800 µM, respectively.

**Table 1: Impact of 5-Thio-L-Fucose treatment on affinity of mAb3 to FcγRIIIa on day 5 (A), 12 (B) and 14 (C). Data represents two biological replicates.**

| **A** | **Day 5** | | | | | |
|---|---|---|---|---|---|---|
| **Sample** | **K_{D} [nM]** | **SEM [nM]** | **Fold change** | **% Fucosylate d** | **% Afucosylat ed** | **% fucose analogue** |
| | | | | | | |
| **control** | 102 | 1 | **1** | 86.5 | 12.4 | 0 |
| **50 µM ThioFuc** | 85 | 2 | **1.2** | 81.4 | 17.7 | 0.38 |
| **200 µM ThioFuc** | 52 | 6 | **2.0** | 62.9 | 22.1 | 14.5 |
| **400 µM ThioFuc** | 35 | 1 | **2.9** | 46.4 | 28.7 | 26.5 |
| **800 µM ThioFuc** | 40 | 3 | **2.6** | 40.4 | 32.3 | 31.7 |
| **400 µM 2F-PerAcFuc** | 78 | 6 | **1.3** | 83.9 | 14.9 | 0.3 |
| | | | | | | |

| **B** | **Day 12** | | | | | |
|---|---|---|---|---|---|---|
| **Sample** | **K_{D} [nM]** | **SEM [nM]** | **Fold change** | **% Fucosylate d** | **% Afucosyl ated** | **% fucose analogue** |
| **control** | 123 | 4 | 1.0 | 90.0 | 9.1 | 0.0 |
| **50 µM ThioFuc** | 63 | 18 | **2.0** | 72.5 | 16.0 | 10.7 |
| **200 µM ThioFuc** | 40 | 11 | **3.1** | 33.3 | 30.1 | 35.9 |
| **400 µM ThioFuc** | 32 | 4 | **3.8** | 20.9 | 34.8 | 44.1 |
| **800 µM ThioFuc** | 25 | 12 | **4.8** | 15.4 | 36.4 | 47.8 |
| **400 µM 2F-PerAcFuc** | 34 | 6 | **3.6** | 64.8 | 34.2 | 0.2 |

| **C** | **Day 14** | | | | | |
|---|---|---|---|---|---|---|
| **Sample** | **K_{D} [nM]** | **SEM [nM]** | **Fold change** | **% Fucosylate d** | **% Afucosyl ated** | **% fucose analogue** |
| **control** | 101 | 31 | **1.0** | 90.1 | 9.6 | 0.0 |
| **50 µM ThioFuc** | 61 | 8 | **1.7** | 69.9 | 16.1 | 13.6 |
| **200 µM ThioFuc** | 28 | 5 | **3.6** | 28.6 | 32.1 | 39.1 |
| **400 µM ThioFuc** | 25 | 3 | **4.1** | 17.7 | 36.0 | 46.2 |
| **800 µM ThioFuc** | 22 | 3 | **4.5** | 12.5 | 38.0 | 49.3 |
| **400 µM 2F-PerAcFuc** | 23 | 3 | **4.4** | 57.4 | 40.9 | 0.12 |

Fig 11 shows the overlay plot of FcγRIIIa binding to mAb3 control (black) or binding to 5-Thio-L-Fucose treated mAb3 (grey) on day 12. Sensorgram shows binding level of a concentration series from 6 to 486 nM to captured FcγRIIIa (n=3). All sensorgrams were double referenced.

## Claims

1. A method of making an antibody or other Fc containing protein having reduced fucosylation, comprising culturing a host cell in a culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-Fucose, 5-alkynyl-5-thio-L-fucose, 6, 6, 6-Trifluoro-5-thio-L-fucose and 5-Thio-L-Fucose phosphonate, wherein the host cell expresses the antibody or other Fc containing protein having a Fc domain having at least one N-glycoside- linked sugar chain bound to the Fc domain through an N-acetylglucosamine of the reducing terminal of the sugar chain, and isolating the antibody or other Fc containing protein from the cells, wherein the antibody or other Fc containing protein has reduced fucosylation at the sugar chain compared to the antibody or other Fc containing protein from the same host cell cultured under the otherwise same culturing conditions but in the absence of 5-Thio-L-fucose and/or a derivative thereof.

2. Method according to claim 1, **characterized in that** the culture medium comprises 5-Thio-L-fucose and/or partially or fully acetylated 5-Thio-L-fucose.

3. Method according to claim 1 or claim 2, **characterized in that** the host cell is a Chinese hamster ovary cell.

4. Method according to one or more of claims 1 to 3, **characterized in that** the host cell is cultured in a fed batch cell culture or a perfusion cell culture.

5. Method according to one or more of claims 1 to 4, **characterized in that** the host cell is cultured by
- Filling into a bioreactor the host cells and an aqueous cell culture medium
- Incubating the cells in the bioreactor
- Continuously over whole time of the incubation of the cells in the bioreactor or once or several times within said incubation time adding a cell culture medium to the bioreactor, whereby the cell culture medium comprises 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-Fucose phosphonate.

6. Method according to one or more of claims 1 to 5, **characterized in that** the medium that is added has a pH below pH 8.5 and comprises 5-Thio-L-fucose and/or a derivative thereof in a concentration between 10 nM and and 100 mmol/l.

7. A method for making a thiofucosylated antibody or other Fc containing protein comprising culturing a host cell in a culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-Fucose, 5-Alkynyl-5-thio-L-fucose, 6, 6, 6-Trifluoro-5-thio-L-fucose and 5-Thio-L-fucose phosphonate, wherein the host cell expresses the antibody or other Fc containing protein having an Fc domain having at least one N-glycoside- linked sugar chain bound to the Fc domain through an N-acetylglucosamine of the reducing terminal of the sugar chain, and isolating the antibody or other Fc containing protein from the cells.

8. A dry powder cell culture medium comprising 5-Thio-L-fucose and/or a 5-Thio-L-fucose derivative selected from partially or fully acetylated 5-Thio-L-fucose, non-, partially or fully acetylated forms of 2-F-5-Thio-L-fucose, 5-Alkynyl-5-thio-L-fucose, 6, 6, 6-Trifluoro-5-thio-L-fucose and 5-Thio-L-fucose phosphonate.

9. Cell culture medium according to claim 8, **characterized in that** the cell culture medium is a feed medium.

10. Cell culture medium according to claims 8 or 9, **characterized in that** the cell culture medium comprises 5-Thio-L-fucose and/or a derivative thereof in an amount that when being dissolved to provide a liquid medium the concentration of the 5-Thio-L-fucose in the liquid medium is between 10 nM and 100 mmol/l.

11. Cell culture medium according to one or more of claims 8 to 10, **characterized in that** the cell culture medium comprises at least one or more saccharide components, one or more amino acids, one or more vitamins or vitamin precursors, one or more salts, one or more buffer components, one or more co-factors and one or more nucleic acid components.

12. A method for producing a cell culture medium according to one or more of claims 8 to 11 by
a) mixing 5-Thio-L-fucose and/or a 5-Thio-L-fucose derivative selected from partially or fully acetylated 5-Thio-L-fucose, non-, partially or fully acetylated forms of 2-F-5-Thio-L-Fucose, 5-alkynyl-5-thio-L-fucose, 6, 6, 6-Trifluoro-5-thio-L-fucose and 5-Thio-L-Fucose phosphonate with the other components of the cell culture medium
b) subjecting the mixture of step a) to milling

13. A method for producing a cell culture medium according to claim 12, **characterized in that** step b) is performed in a pin mill, fitz mill or a jet mill.

14. A method for producing a cell culture medium according to claim 12 or 13, **characterized in that** the mixture from step a) is cooled to a temperature below 0°C prior to milling.

## Patentansprüche

1. Verfahren zur Herstellung eines Antikörpers oder eines anderen Fc-haltigen Proteins mit reduzierter Fucosylierung, umfassend das Züchten einer Wirtszelle in einem Kulturmedium, das 5-Thio-L-fucose und/oder ein 5-Thio-L-fucose-Derivat, das ausgewählt ist aus teilweise oder vollständig acetylierter 5-Thio-L-fucose, nicht, teilweise oder vollständig acetylierten Formen von 2-F-5-Thio-L-fucose, 5-Alkinyl-5-thio-L-fucose, 6,6,6-Trifluor-5-thio-L-fucose und 5-Thio-L-fucosephosphonat, enthält, wobei die Wirtszelle den Antikörper oder das andere Fc-haltige Protein mit einer Fc-Domäne, die mindestens eine N-Glycosid-verknüpfte Zuckerkette aufweist, die über ein N-Acetylglucosamin des reduzierenden Endes der Zuckerkette an die Fc-Domäne gebunden ist, exprimiert, und das Isolieren des Antikörpers oder des anderen Fc-haltigen Proteins aus den Zellen, wobei der Antikörper oder das andere Fc-haltige Protein eine verringerte Fucosylierung an der Zuckerkette aufweist, verglichen mit dem Antikörper oder dem anderen Fc-haltigen Protein aus der gleichen Wirtszelle, die unter den ansonsten gleichen Kulturbedingungen, aber in Abwesenheit von 5-Thio-L-fucose und/oder einem Derivat davon gezüchtet wurde.

2. Verfahren nach Anspruch 1, **dadurch gekennzeichnet, dass** das Kulturmedium 5-Thio-L-fucose und/oder teilweise oder vollständig acetylierte 5-Thio-L-fucose enthält.

3. Verfahren nach Anspruch 1 oder Anspruch 2, **dadurch gekennzeichnet, dass** die Wirtszelle eine Ovarialzelle des chinesischen Hamsters ist.

4. Verfahren nach einem oder mehreren der Ansprüche 1 bis 3, **dadurch gekennzeichnet, dass** die Wirtszelle in einer Fed-Batch-Zellkultur oder einer Perfusionszellkultur gezüchtet wird.

5. Verfahren nach einem oder mehreren der Ansprüche 1 bis 4, **dadurch gekennzeichnet, dass** die Wirtszelle gezüchtet wird durch
- Einfüllen der Wirtszellen und eines wässrigen Zellkulturmediums in einen Bioreaktor
- Inkubieren der Zellen in dem Bioreaktor
- Kontinuierliches Zugeben eines Zellkulturmediums in den Bioreaktor über die gesamte Zeit der Inkubation der Zellen im Bioreaktor oder ein- oder mehrmaliges Zugeben innerhalb dieser Inkubationszeit, wobei das Zellkulturmedium 5-Thio-L-fucose und/oder ein 5-Thio-L-fucose-Derivat enthält, das ausgewählt ist aus teilweise oder vollständig acetylierter 5-Thio-L-fucose, nicht, teilweise oder vollständig acetylierten Formen von 2-F-5-Thio-L-fucose, 5-Alkinyl-5-thio-L-fucose, 6,6,6-Trifluor-5-thio-L-fucose und 5-Thio-L-fucosephosphonat.

6. Verfahren nach einem oder mehreren der Ansprüche 1 bis 5, **dadurch gekennzeichnet, dass** das zugegebene Medium einen pH-Wert unter pH 8,5 aufweist und 5-Thio-L-fucose und/oder ein Derivat davon in einer Konzentration zwischen 10 nM und 100 mmol/l enthält.

7. Verfahren zur Herstellung eines thiofucosylierten Antikörpers oder eines anderen Fc-haltigen Proteins, umfassend das Züchten einer Wirtszelle in einem Kulturmedium, das 5-Thio-L-fucose und/oder ein 5-Thio-L-fucose-Derivat, das ausgewählt ist aus teilweise oder vollständig acetylierter 5-Thio-L-fucose, nicht, teilweise oder vollständig acetylierten Formen von 2-F-5-Thio-L-fucose, 5-Alkinyl-5-thio-L-fucose, 6,6,6-Trifluor-5-thio-L-fucose und 5-Thio-L-fucosephosphonat, enthält, wobei die Wirtszelle den Antikörper oder das andere Fc-haltige Protein mit einer Fc-Domäne, die mindestens eine N-Glycosid-verknüpfte Zuckerkette aufweist, die über ein N-Acetylglucosamin des reduzierenden Endes der Zuckerkette an die Fc-Domäne gebunden ist, exprimiert, und das Isolieren des Antikörpers oder des anderen Fc-haltigen Proteins aus den Zellen.

8. Trockenpulver-Zellkulturmedium, enthaltend 5-Thio-L-fucose und/oder ein 5-Thio-L-fucose-Derivat, das ausgewählt ist aus teilweise oder vollständig acetylierter 5-Thio-L-fucose, nicht, teilweise oder vollständig acetylierten Formen von 2-F-5-Thio-L-fucose, 5-Alkinyl-5-thio-L-fucose, 6,6,6-Trifluor-5-thio-L-fucose und 5-Thio-L-fucosephosphonat.

9. Zellkulturmedium nach Anspruch 8, **dadurch gekennzeichnet, dass** das Zellkulturmedium ein Zufütterungsmedium ist.

10. Zellkulturmedium nach den Ansprüchen 8 oder 9, **dadurch gekennzeichnet, dass** das Zellkulturmedium 5-Thio-L-fucose und/oder ein Derivat davon in einer Menge enthält, dass, wenn es gelöst wird, um ein flüssiges Medium bereitzustellen, die Konzentration der 5-Thio-L-fucose in dem flüssigen Medium zwischen 10 nM und 100 mmol/l liegt.

11. Zellkulturmedium nach einem oder mehreren der Ansprüche 8 bis 10, **dadurch gekennzeichnet, dass** das Zellkulturmedium mindestens eine oder mehrere Saccharidkomponenten, eine oder mehrere Aminosäuren, ein(e) oder mehrere Vitamine oder Vitaminvorstufen, ein oder mehrere Salze, eine oder mehrere Pufferkomponenten, einen oder mehrere Co-Faktoren und eine oder mehrere Nukleinsäurekomponenten enthält.

12. Verfahren zur Herstellung eines Zellkulturmediums nach einem oder mehreren der Ansprüche 8 bis 11, indem man
a) 5-Thio-L-fucose und/oder ein 5-Thio-L-fucose-Derivat, das ausgewählt ist aus teilweise oder vollständig acetylierter 5-Thio-L-fucose, nicht, teilweise oder vollständig acetylierten Formen von 2-F-5-Thio-L-fucose, 5-Alkinyl-5-thio-L-fucose, 6,6,6-Trifluor-5-thio-L-fucose und 5-Thio-L-fucosephosphonat, mit den anderen Komponenten des Zellkulturmediums mischt
b) die Mischung aus Schritt a) einem Mahlen unterwirft.

13. Verfahren zur Herstellung eines Zellkulturmediums nach Anspruch 12, **dadurch gekennzeichnet, dass** Schritt b) in einer Stiftmühle, Fitzmühle oder einer Strahlmühle durchgeführt wird.

14. Verfahren zur Herstellung eines Zellkulturmediums nach Anspruch 12 oder 13, **dadurch gekennzeichnet, dass** die Mischung aus Schritt a) vor dem Mahlen auf eine Temperatur unter 0°C gekühlt wird.

## Revendications

1. Méthode de préparation d'un anticorps ou d'une autre protéine contenant un fragment Fc ayant une fucosylation réduite, comprenant la culture d'une cellule hôte dans un milieu de culture comprenant du 5-thio-L-fucose et/ou un dérivé de 5-thio-L-fucose choisi parmi le 5-thio-L-fucose partiellement ou complètement acétylé, les formes non, partiellement ou complètement acétylées de 2-F-5-thio-L-fucose, de 5-alcynyl-5-thio-L-fucose, de 6,6,6-trifluoro-5-thio-L-fucose et de phosphonate de 5-thio-L-fucose, où la cellule hôte exprime l'anticorps ou l'autre protéine contenant un fragment Fc ayant un domaine Fc ayant au moins une chaîne de sucre liée à un N-glycoside fixée au domaine Fc via une N-acétylglucosamine de l'extrémité terminale réductrice de la chaîne de sucre, et l'isolement de l'anticorps ou de l'autre protéine contenant un fragment Fc à partir des cellules, où l'anticorps ou l'autre protéine contenant un fragment Fc présente une fucosylation réduite au niveau de la chaîne de sucre par rapport à l'anticorps ou à l'autre protéine contenant un fragment Fc provenant de la même cellule hôte cultivée dans des conditions de culture par ailleurs semblables mais en l'absence de 5-thio-L-fucose et/ou d'un dérivé de celui-ci.

2. Méthode selon la revendication 1, **caractérisée en ce que** le milieu de culture comprend du 5-thio-L-fucose et/ou du 5-thio-L-fucose partiellement ou complètement acétylé.

3. Méthode selon la revendication 1 ou la revendication 2, **caractérisée en ce que** la cellule hôte est une cellule ovarienne de hamster chinois.

4. Méthode selon l'une ou plusieurs parmi les revendications 1 à 3, **caractérisée en ce que** la cellule hôte est cultivée dans une culture cellulaire discontinue renouvelée ou dans une culture cellulaire en perfusion.

5. Méthode selon l'une ou plusieurs parmi les revendications 1 à 4, **caractérisée en ce que** la cellule hôte est cultivée par
- le chargement, dans un bioréacteur, des cellules hôtes et d'un milieu de culture cellulaire aqueux,
- l'incubation des cellules dans le bioréacteur,
- de manière continue, sur toute la durée de l'incubation des cellules dans le bioréacteur ou une ou plusieurs fois au cours dudit temps d'incubation, l'addition d'un milieu de culture cellulaire au bioréacteur, où le milieu de culture cellulaire comprend du 5-thio-L-fucose et/ou un dérivé de 5-thio-L-fucose choisi parmi le 5-thio-L-fucose partiellement ou complètement acétylé, les formes non, partiellement ou complètement acétylées de 2-F-5-thio-L-fucose, de 5-alcynyl-5-thio-L-fucose, de 6,6,6-trifluoro-5-thio-L-fucose et de phosphonate de 5-thio-L-fucose.

6. Méthode selon l'une ou plusieurs parmi les revendications 1 à 5, **caractérisée en ce que** le milieu qui est ajouté présente un pH inférieur à pH 8,5 et comprend du 5-thio-L-fucose et/ou un dérivé de celui-ci selon une concentration comprise entre 10 nM et 100 mmol/l.

7. Méthode de préparation d'un anticorps thiofucosylé ou d'une autre protéine contenant un fragment Fc, comprenant la culture d'une cellule hôte dans un milieu de culture comprenant du 5-thio-L-fucose et/ou un dérivé de 5-thio-L-fucose choisi parmi le 5-thio-L-fucose partiellement ou complètement acétylé, les formes non, partiellement ou complètement acétylées de 2-F-5-thio-L-fucose, de 5-alcynyl-5-thio-L-fucose, de 6,6,6-trifluoro-5-thio-L-fucose et de phosphonate de 5-thio-L-fucose, où la cellule hôte exprime l'anticorps ou l'autre protéine contenant un fragment Fc ayant un domaine Fc ayant au moins une chaîne de sucre liée à un N-glycoside fixée au domaine Fc via une N-acétylglucosamine de l'extrémité terminale réductrice de la chaîne de sucre, et l'isolement de l'anticorps ou de l'autre protéine contenant un fragment Fc à partir des cellules.

8. Milieu de culture cellulaire en poudre sèche comprenant du 5-thio-L-fucose et/ou un dérivé de 5-thio-L-fucose choisi parmi le 5-thio-L-fucose partiellement ou complètement acétylé, les formes non, partiellement ou complètement acétylées de 2-F-5-thio-L-fucose, de 5-alcynyl-5-thio-L-fucose, de 6,6,6-trifluoro-5-thio-L-fucose et de phosphonate de 5-thio-L-fucose.

9. Milieu de culture cellulaire selon la revendication 8, **caractérisé en ce que** le milieu de culture cellulaire est un milieu d'alimentation.

10. Milieu de culture cellulaire selon les revendications 8 ou 9, **caractérisé en ce que** le milieu de culture cellulaire comprend du 5-thio-L-fucose et/ou un dérivé de celui-ci selon une quantité telle que, lors d'une solubilisation afin de fournir un milieu liquide, la concentration en 5-thio-L-fucose dans le milieu liquide est comprise entre 10 nM et 100 mmol/l.

11. Milieu de culture cellulaire selon l'une ou plusieurs parmi les revendications 8 à 10, **caractérisé en ce que** le milieu de culture cellulaire comprend au moins un ou plusieurs composants saccharidiques, un ou plusieurs acides aminés, un(e) ou plusieurs vitamines ou précurseurs de vitamines, un ou plusieurs sels, un ou plusieurs composants de tampon, un ou plusieurs cofacteurs et un ou plusieurs composants d'acides nucléiques.

12. Méthode de production d'un milieu de culture cellulaire selon l'une ou plusieurs parmi les revendications 8 à 11, par
a) le mélange de 5-thio-L-fucose et/ou d'un dérivé de 5-thio-L-fucose choisi parmi le 5-thio-L-fucose partiellement ou complètement acétylé, les formes non, partiellement ou complètement acétylées de 2-F-5-thio-L-fucose, de 5-alcynyl-5-thio-L-fucose, de 6,6,6-trifluoro-5-thio-L-fucose et de phosphonate de 5-thio-L-fucose avec les autres composants du milieu de culture cellulaire,
b) la soumission du mélange de l'étape a) à un broyage.

13. Méthode de production d'un milieu de culture cellulaire selon la revendication 12, **caractérisée en ce que** l'étape b) est mise en oeuvre dans un broyeur à broches, un broyeur FitzMill ou un broyeur à jet.

14. Méthode de production d'un milieu de culture cellulaire selon la revendication 12 ou 13, **caractérisée en ce que** le mélange de l'étape a) est refroidi jusqu'à une température inférieure à 0°C préalablement au broyage.
